# EUROPÄISCHE PATENTSCHRIFT

(11) **EP 0 804 507 B1**
(45) Veröffentlichungstag und Bekanntmachung des Hinweises auf die Patenterteilung: **28.04.1999**
(21) Anmeldenummer: 96900944.8
(22) Anmeldetag: 12.01.1996
(51) Int. Cl.: C09B 5/62, C07D 221/18

(54) **SUBSTITUIERTE QUATERRYLENTETRACARBONSÄUREDIIMIDE**
SUBSTITUTED QUATERRYLENE TETRACARBOXYLIC ACID DIIMIDES
DIIMIDES DE QUATERRYLENE ACIDE TETRACARBOXYLIQUE SUBSTITUES

(30) Priorität: 20.01.1995 DE 19501576
(43) Veröffentlichungstag der Anmeldung: 05.11.1997
(73) Patentinhaber: BASF AKTIENGESELLSCHAFT, 67056 Ludwigshafen (DE); Max-Planck-Gesellschaft zur Förderung der Wissenschaften e.V. Berlin, 80539 München (DE)
(72) Erfinder: MÜLLEN, Klaus, D-50939 Köln (DE); QUANTE, Heribert, D-50823 Köln (DE); BÖHM, Arno, D-68305 Mannheim (DE)
(86) Internationale Anmeldenummer: EP9600118
(87) Internationale Veröffentlichungsnummer: WO9622332

(56) Entgegenhaltungen:
- DE-A- 4 236 885
- DYES AND PIGMENTS, Bd. 16, Nr. 1, 1.Januar 1991, Seiten 19-25, XP000208007 NAGAO Y ET AL: "SYNTHESIS AND PROPERTIES OF N-ALKYLBROMOPERYLENE-3,4-DICARBOXIMIDES"
- ANGEWANDTE CHEMIE INTERNATIONAL EDITION., Bd. 34, Nr. 12, 7.Juli 1995, WEINHEIM DE, Seiten 1323-1325, XP002002793 H.QUANTE ET AL.: "Quaterrylenebis(dicarboximides)"
- DYES AND PIGMENTS, Bd. 11, Nr. 4, 1.Januar 1989, Seiten 303-317, XP000084462 SEYBOLD G ET AL: "NEW PERYLENE AND VIOLANTHRONE DYESTUFFS FOR FLUORESCENT COLLECTORS"

## Beschreibung

Die vorliegende Erfindung betrifft neue Quaterrylentetracarbonsäurediimide der allgemeinen Formel I 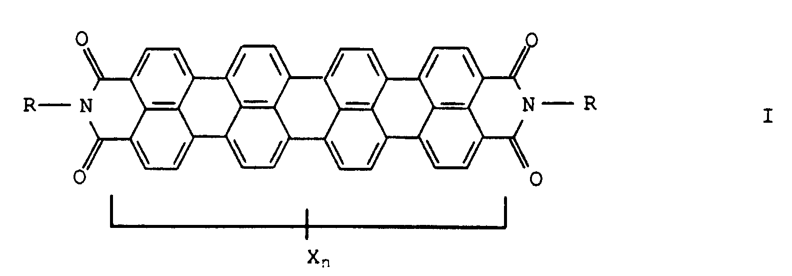 in der die Variablen folgende Bedeutung haben:
- R: Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann, wobei
R¹ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR¹- unterbrochen sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Halogen substituiert sein kann, ein- oder mehrfach substituiert sein kann, wobei
R² Wasserstoff; C₁-C₁₈-Alkyl; Aryl oder Hetaryl, das jeweils durch C₁-C₆-Alkyl, C₁-C₆-Alkoxy, Halogen oder Cyano substituiert sein kann, bedeutet;
- X: Halogen; C₁-C₁₈-Alkyl; Aryloxy, Arylthio, Hetaryloxy oder Hetarylthio, das jeweils durch C₁-C₄-Alkyl oder C₁-C₄-Alkoxy substituiert sein kann;
- n: eine Zahl von 2 bis 12.

Außerdem betrifft die Erfindung die Herstellung dieser Quaterrylentetracarbonsäurediimide und ihre Verwendung als Fluoreszenzfarbstoffe oder Pigmente.

Nicht zuletzt betrifft die Erfindung neue 9-Halogenperylen-3,4-dicarbonsäureimide der allgemeinen Formel III 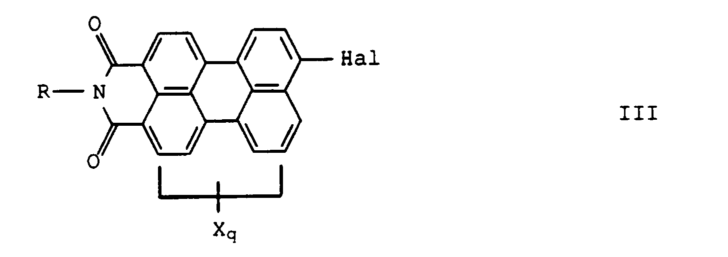 in der R und X die oben angegebene Bedeutung haben und Hal Halogen und q 2 bis 4 bedeutet, als Zwischenprodukte für die Quaterrylentetracarbonsäurediimide I.

In der EP-A-596 292 wird am Beispiel von N,N'-Didodecylquaterrylen-3,4:13,14-tetracarbonsäurediimid die Herstellung unsubstituierter Quaterrylentetracarbonsäurediimide (Formel I: n = 0; R = n-C₁₂H₂₅) und ihre Eignung als Fluoreszenzfarbstoffe und Pigmente beschrieben.

Ausgehend von N-Dodecylperylen-3,4,9,10-tetracarbonsäure-3,4-imid-9,10-anhydrid, wird durch Decarboxylierung in Kalilauge unter Druck und bei erhöhter Temperatur N-Dodecylperylen-3,4-dicarbonsäureimid erhalten, welches zu N-Dodecyl-9-bromperylen-3,4-dicarbonsäureimid bromiert wird. Dieses wird unter Bromabspaltung in Gegenwart eines inerten Verdünnungsmittels und eines metallorganischen Katalysators zum entsprechenden Biperylenderivat umgesetzt, welches schließlich durch Erhitzen in alkalischem Medium in Gegenwart eines Oxidationsmittels in das o.g. Quaterrylenderivat überführt wird.

In der EP-A-596 292 wird jedoch kein Weg zur Herstellung substituierter Quaterrylentetracarbonsäurediimide I (im folgenden kurz "Quaterrylimide" genannt) offenbart, die aber gerade von besonderem Interesse sind, da durch gezielte Substitution die anwendungstechnischen Eigenschaften (z.B. Löslichkeit, Hydrophilie bzw. Lipophilie, Absorptions- und Emissionsverhalten) eingestellt und variiert werden können.

Der Erfindung lag daher die Aufgabe zugrunde, neue, substituierte Quaterrylimide bereitzustellen, die sich durch günstige Anwendungseigenschaften (bei Fluoreszenzfarbstoffen beispielsweise auch gute Löslichkeit in den Anwendungsmedien) auszeichnen, und damit die Möglichkeit zur optimalen Anpassung an den jeweiligen Anwendungszweck zu bieten.

Demgemäß wurden die Quaterrylimide der eingangs definierten Formel I gefunden.

Bevorzugte Quaterrylimide I sind den Unteransprüchen zu entnehmen.

Außerdem wurden verschiedene Verfahren zur Herstellung der Quaterrylimide I gefunden, welche ebenfalls den Ansprüchen zu entnehmen sind.

Schließlich wurde die Verwendung der Quaterrylimide I als Pigmente oder Fluoreszenzfarbstoffe gefunden.

Nicht zuletzt wurden die 9-Halogenperylen-3,4-dicarbonsäureimide der eingangs definierten Formel III als Zwischenprodukte für die Herstellung der Quaterrylimide I gefunden.

Alle in den Formeln I auftretenden Alkylgruppen können sowohl geradkettig als auch verzweigt sein. Aromatische Reste, die substituiert sind, können im allgemeinen bis zu 3, bevorzugt 1 oder 2 der genannten Substituenten aufweisen.

Als Beispiele für geeignete Reste R und X (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl, 3, 6, 9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3-Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazoaoctyl,3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2-Hydroxypropyl, 1-Hydroxyprop-2-yl, 2- und 4-Hydroxybutyl, 1-Hydroxybut-2-yl und 8-Hydroxy-4-oxaoctyl, 2-Cyanoethyl, 3-Cyanopropyl, 2-Methyl-3-ethyl-3-cyanopropyl, 7-Cyano-7-ethylheptyl und 4-Methyl-7-methyl-7-cyanoheptyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Formylamino, Acetylamino, Propionylamino und Benzoylamino; Chlor, Brom und Iod;
Phenylazo, 2-Naphthylazo, 2-Pyridylazo und 2-Pyrimidylazo;
Cyclopentyl, Cyclohexyl, Cycloheptyl, Cyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl;
Phenyl, 2-Naphthyl, 2- und 3-Pyrryl, 2-, 3- und 4-Pyridyl, 2-, 4-und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1-und 5-Isochinolyl;
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl, 2-, 3- und 4-Isobutylphenyl, 2,4-, 3,5 und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3-und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3- und 4-Hydroxyphenyl und 2,4-, 3,5- und
2,6-Dihydroxyphenyl; 2-, 3- und 4-Cyanophenyl; 3- und 4-Carboxyphenyl; 3- und 4-Carboxamidophenyl, 3- und 4-N-(Methyl)carboxamidophenyl und 3- und 4-N-(Ethyl)carboxamidophenyl; 3- und 4-Acetylaminophenyl, 3- und 4-Propionylaminophenyl und 3- und 4-Butyrylaminophenyl; 3- und 4-N-(Phenyl)aminophenyl, 3- und 4-(N-(o-Tolyl))-aminophenyl, 3- und 4-(N-(m-Tolyl))aminophenyl und 3- und 4-(N-(p-Tolyl))aminophenyl; 3- und 4-(2-Pyridyl)aminophenyl, 3- und 4-(3-Pyridyl)aminophenyl, 3- und 4-(4-Pyridyl)aminophenyl, 3-und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Bei den erfindungsgemäßen Verfahren zur Herstellung der kernsubstituierten Quaterrylimide I wird stets von einem Perylen-3,4-dicarbonsäureimid II (im folgenden kurz "Perylimid" genannt) ausgegangen. In Abhängigkeit davon, welches Substitutionsmuster die Quaterrylimide I aufweisen sollen, ist entweder ein unsubstituiertes oder ein bereits am Perylenkern substituiertes Perylen-3,4-dicarbonsäureimid II als Ausgangsmaterial geeignet.

Quaterrylimide der Formel Ia 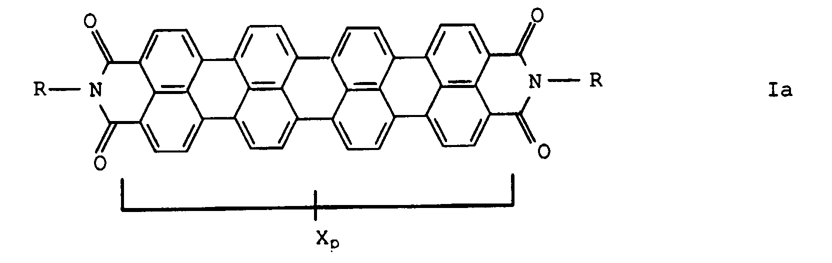 die 4 oder 8 der Substituenten X tragen und zwar in den Positionen 1,7,10,16 und 1,7,11,17 bzw. 1,6,7,10,11,16,17,20, werden vorteilhaft ausgehend von substituierten Perylimiden der Formel IIa 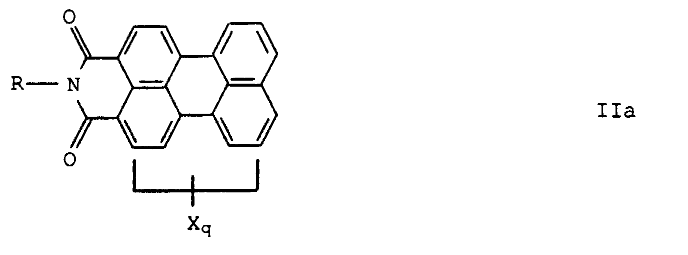 die bereits 2 oder 4 der Substituenten X tragen und zwar in den Positionen 1,7 bzw. 1,6,7,12, erhalten.

Quaterrylimide der Formel Ib 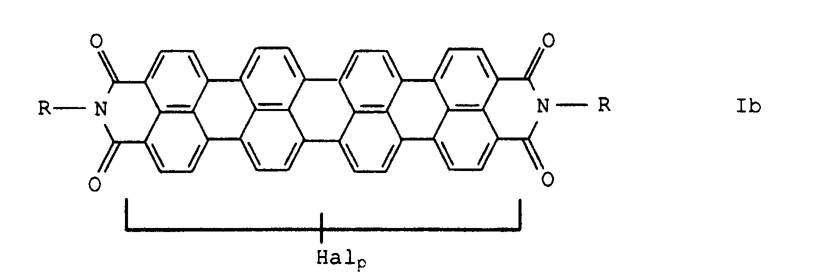 die 4 oder 8 Halogenatome (bevorzugt Chlor oder Brom) enthalten und zwar in den Positionen 1,6,11,16 bzw. 1,6,7,10,11,16,17,20, werden erfindungsgemäß durch selektive Halogenierung der unsubstituierten Perylimide der Formel IIb 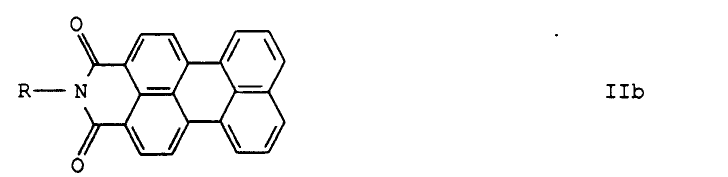 erhalten. Die Zahl von 8 Substituenten wird insbesondere bei chlorierten Produkten erreicht, die selbstverständlich auch nur 4 Chloratome enthalten können, während durch Bromierung in der Regel nur eine Substituentenzahl von 4 erzielt wird, jeweils (ganz besonders bei der Bromierung) selektiv in den genannten Positionen.

Quaterrylimide der Formel Ic 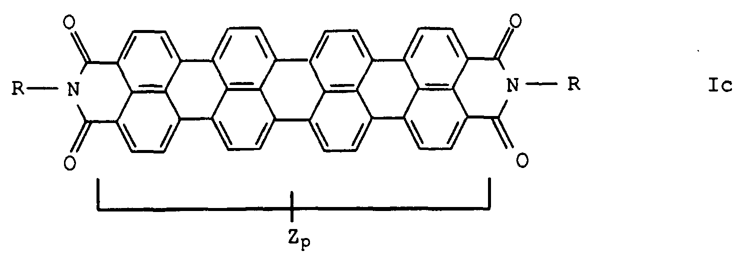 die durch 4 oder 8 Substituenten Z (Aryloxy, Arylthio, Hetaryloxy oder Hetarylthio, das jeweils durch C₁-C₄-Alkyl oder C₁-C₄-Alkoxy substituiert sein kann, vorzugsweise Phenoxy oder Pyrimidyloxy) substituiert sind und zwar in den für die Quaterrylimide Ib genannten Positionen 1,6,11,16 bzw. 1,6,7,10,11,16,17,20, lassen sich erfindungsgemäß ebenfalls von den unsubstituierten Perylen-3,4-dicarbonsäureimiden IIb ausgehend herstellen, indem die Perylimide IIb zunächst halogeniert und dann einer nucleophilen Substitution (Austausch der Halogenatome durch die Reste Z) unterzogen werden.

Quaterrylimide der Formel Id 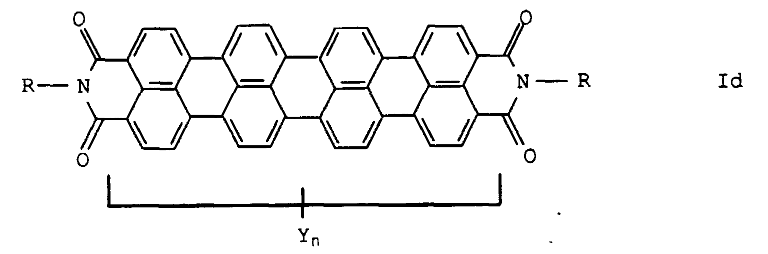 die 2, 4, 6, 8 oder 12 Substituenten Y (Halogen oder Reste Z, wie sie bei den Quaterrylimiden der Formel Ic genannt sind) tragen und zwar bevorzugt in den Positionen 1,6; 1,7,8,18; 1,7,9,11,17,19; 1,6,7,10,11,16,17,20 bzw. 1,6,7,8,9,10,11,16,17,18,19,20, können durch Halogenierung (und gegebenenfalls nucleophilen Austausch) der unsubstituierten Quaterrylimide Id' (n = 0) erhalten werden, die ebenfalls wie die erfindungsgemäßen substituierten Quaterrylimide Ia - Ic ausgehend von entsprechenden Perylimiden II, durch deren Überführung in 9-Halogenperylimide (insbesondere 9-Bromperylimide) III, Kupplung zweier 9-Halogenperylimide zu entsprechenden Biperylenen IV und deren Oxidation zu den Quaterrylimiden I zugänglich sind.

Die bei den erfindungsgemäßen Herstellungsverfahren als Ausgangsstoffe eingesetzten Perylen-3,4-dicarbonsäureimide II sind in der ebenfalls anhängigen deutschen Patentanmeldung 195 01 737.4 beschrieben und können vorteilhaft nach den dort ebenfalls vorgestellten Verfahren hergestellt und gewünschtenfalls gereinigt werden.

Danach erfolgt die Herstellung der Perylimide II durch Umsetzung der entsprechend substituierten Perylen-3,4,9,10-tetracarbonsäuren bzw. deren Anhydride, insbesondere der Dianhydride, mit den gewünschten primären Aminen (RNH₂).

Die Perylen-3,4,9,10-tetracarbonsäuren bzw. deren Anhydride können ihrerseits durch Halogenierung und gewünschtenfalls anschließenden Austausch der Halogenatome durch Aryloxy-, Arylthio-, Hetaryloxy-, Hetarylthio- oder Alkylreste erhalten werden.

Die besonders interessanten, 1,7-disubstituierten Perylen-3,4,9,10-tetracarbonsäuren bzw. Anhydride sind dabei, wie in den älteren deutschen Patentanmeldungen 195 47 209.8 und 195 47 210.1 beschrieben, nach einem mehrstufigen Verfahren ausgehend von durch selektive Bromierung von Perylen-3,4,9,10-tetracarbonsäure bzw. -dianhydrid in 100 gew.-%iger Schwefelsäure bei 80 bis 90°C hergestellter 1,7-Dibromperylen-3,4,9,10-tetracarbonsäure bzw. deren Dianhydrid erhältlich. Diese werden in Gegenwart eines polaren aprotischen Lösungsmittels wie N-Methylpyrrolidon und gegebenenfalls eines Imidierungskatalysators, z.B. einer organischen oder anorganischen Säure oder eines Übergangsmetallsalzes, mit einem primären Amin zum entsprechenden 1,7-Dibromperylen-3,4,9,10-tetracarbonsäurediimid umgesetzt, welches anschließend entweder in Gegenwart eines inerten aprotischen Lösungsmittels wie N-Methylpyrrolidon und einer nicht oder nur schwach nucleophilen Base, z.B. Natrium- oder Kaliumcarbonat, mit einem aromatischen Alkohol oder Thioalkohol oder aber in Gegenwart eines aprotischen Lösungsmittels wie Tetrahydrofuran, eines Palladiumkomplexes als Katalysator sowie eines Kupfersalzes als Cokatalysator und einer Base, z.B. Piperidin, mit einem 1-Alkin umgesetzt wird. Im letztgenannten Fall werden 1,7-disubstituierte Perylen-3,4,9,10-tetracarbonsäurediimide erhalten, die im Substituenten R² ungesättigte Bindungen enthalten, die durch Nachrühren in einer Wasserstoffatmosphäre oder durch katalytische Reduktion mit Wasserstoff reduziert werden können. In einem letzten Reaktionsschritt wird das entweder 1,7-diaroxy-, -diarylthiooder -dialkylsubstituierte Perylen-3,4,9,10-tetracarbonsäurediimid dann in Gegenwart eines polaren protischen Lösungsmittels wie Isopropanol und einer Base, z.B. Natrium- oder Kaliumhydroxid, zu der 1,7-disubstituierten Perylen-3,4,9,10-tetracarbonsäure bzw. deren Dianhydrid verseift.

Die Umsetzung der Perylen-3,4,9,10-tetracarbonsäuren bzw. der Anhydride mit den primären Aminen RNH₂ erfolgt nach dem in der ebenfalls anhängigen deutschen Patentanmeldung 195 01 737.4 beschriebenen Verfahren in Gegenwart einer tertiären stickstoffbasischen Verbindung als Lösungsmittel und eines Übergangsmetalls oder Übergangsmetallsalzes als Katalysator.

Als Beispiele für geeignete Stickstoffbasen werden cyclische Imide wie N-Methylpyrrolidon, tertiäre aliphatische Amine NR³, deren Alkylreste R 4 bis 8 C-Atome aufweisen, wie Trihexylamin, und vor allem aromatische Heterocyclen wie Chinaldin, Isochinolin und insbesondere Chinolin genannt.

Die Lösungsmittelmenge beträgt üblicherweise 2 bis 20 kg, vorzugsweise 6 bis 12 kg, je kg Perylen-3,4,9,10-tetracarbonsäuredianhydrid.

Als Katalysatoren werden insbesondere die Übergangsmetalle Eisen und vor allem Zink und Kupfer sowie besonders auch deren anorganische und organische Salze, vorzugsweise Kupfer(I)oxid, Kupfer(II)oxid, Kupfer(I)chlorid, Kupfer(II)acetat, Zinkacetat und Zinkpropionat, eingesetzt.

In der Regel kommen 5 bis 80 Gew.-% Katalysator, bezogen auf das Perylen-3,4,9,10-tetracarbonsäuredianhydrid, zum Einsatz. Bevorzugte Mengen betragen bei den Kupferverbindungen 10 bis 25 Gew.-% und bei den Zinksalzen 40 bis 60 Gew.-%, ebenfalls bezogen auf das Anhydrid.

Als primäre Amine können bei diesem Verfahren alle bei der Reaktionstemperatur stabilen primären Amine, vorzugsweise solche, deren Siedepunkt beim Reaktionsdruck oberhalb der Reaktionstemperatur liegt, eingesetzt werden.

Die Reaktionstemperatur beträgt im allgemeinen 120 bis 250°C, insbesondere 170 bis 235°C, wobei zweckmäßigerweise unter Schutzgas (z.B. Stickstoff) sowohl bei Normaldruck als auch bei einem Überdruck von üblicherweise bis zu 10 bar gearbeitet wird.

Das dort verwendete Molverhältnis der Ausgangsverbindungen Amin und Anhydrid liegt in der Regel bei 0,8:1 bis 6:1. Bei druckloser Fahrweise (d.h. Normaldruck) beträgt es vorzugsweise 0,8:1 bis 1,2:1, bei Umsetzung unter erhöhtem Druck insbesondere 2:1 bis 4:1.

Genügen die nach diesem Verfahren erhaltenen (Roh-)Produkte nicht den gewünschten Reinheitsanforderungen für die vorliegende Umsetzung (in der Regel werden Produkte mit Wertgehalten > 80 % erhalten), so können sie noch dem ebenfalls beschriebenen Reinigungsverfahren unterzogen werden.

Dabei werden die Perylen-3,4-dicarbonsäureimid-Rohprodukte zunächst durch etwa 10 bis 60 minütiges Erhitzen in der üblicherweise 3- bis 10fachen, vorzugsweise 5,5- bis 6,5fachen, Gewichtsmenge N-Methylpyrrolidon (kurz: NMP) in NMP-Addukte überführt.

Geeignete Temperaturen betragen hierbei im allgemeinen 140 bis 200°C, bevorzugt 160 bis 180°C.

Erfolgte die Herstellung des Imids bereits in NMP als Lösungsmittel, kann dieser Schritt selbstverständlich entfallen.

Die isolierten NMP-Addukte werden anschließend einer alkalischen Reinigungsbehandlung in Gegenwart eines organischen Verdünnungsmittels bei etwa 50 bis 150°C, bevorzugt 60 bis 120°C, unterzogen. Gewünschtenfalls kann noch eine saure Nachbehandlung angeschlossen werden.

Als Verdünnungsmittel eignen sich dabei neben inerten aromatischen Lösungsmitteln wie Toluol und Xylol vorzugsweise Alkohole, die ein- oder mehrwertig sein können, z.B. aromatische Alkohole wie Phenol und Benzylalkohol und aliphatische Alkohole, sowohl Glykole und Glykolether, wie Ethylenglykol, Propylenglykol und Butylglykol als auch insbesondere C₂-C₆-Alkohole wie Ethanol, Propanol, Butanol, Pentanol und Hexanol, die auch verzweigt sein können, wie bevorzugt Isopropanol.

In der Regel werden 40 bis 200 kg, insbesondere 80 bis 120 kg Verdünnungsmittel je kg NMP-Addukt eingesetzt.

Geeignete Basen sind neben sterisch gehinderten Stickstoffbasen, wie Diazabicycloundecen und Diazabicyclo[2.2.2]octan, vor allem Alkalimetallhydroxide, wie Natriumhydroxid und insbesondere Kaliumhydroxid, die zweckmäßigerweise im Gemisch mit Wasser verwendet werden, und Alkalimetallsalze sekundärer und tertiärer aliphatischer (bevorzugt C₃-C₆-)Alkohole, wie Natrium-tert.-butylat und insbesondere Kalium-tert.-butylat.

Üblicherweise kommen je kg NMP-Addukt 2 bis 15 kg Base zum Einsatz, vorzugsweise 5 bis 7 kg, insbesondere etwa 6 kg, trockenes Alkalimetallhydroxid bzw. 0,5 bis 1,5 kg, insbesondere 0,7 bis 1 kg, Alkoholat oder Stickstoffbase.

Gewünschtenfalls kann man noch eine zusätzliche Säurebehandlung anschließen, bei der man den nicht getrockneten Filterkuchen in einer verdünnten anorganischen Säure, z.B. 4 bis 6 gew.-%iger Salzsäure, (etwa 4 bis 6 kg Säure je kg Filterkuchen) aufrührt, und das gereinigte Imid II danach wie üblich isolieren.

Bei allen erfindungsgemäßen Herstellungsvarianten besteht der erste Verfahrensschritt a) darin, das als Edukt dienende Perylimid II zu halogenieren. Je nach den gewünschten Quaterrylimiden I wird hierbei nur die 9-Position des Perylimids halogeniert (vorzugsweise bromiert) (in den Fällen Ia und Id) oder werden zusätzlich zu der 9-Position 2 oder 4 weitere Positionen (1- und 6-Position bzw. 1-, 6-, 7- und 12-Position; syn-Halogenierung) halogeniert (in den Fällen Ib und Ic) und so die halogenierten Perylimide der Formeln IIIa, IIIb und IIId erhalten.

Von besonderer Bedeutung ist, daß die Substituenten R und X nicht von Halogen angegriffen werden.

Die erfindungsgemäße Halogenierung a) wird stets in Gegenwart eines inerten Verdünnungsmittels durchgeführt.

Geeignete Verdünnungsmittel sind neben halogenierten Aromaten wie Chlor-, Dichlor- und Trichlorbenzolen beispielsweise Halogenkohlenwasserstoffe, vor allem Methane und Ethane, wie Tribrom-, Tetrachlor- und Tetrabrommethan, 1,2-Dichlor-, 1,1-Dibrom-, 1,2-Dibrom-, 1,1,1-Trichlor-, 1,1,2-Trichlor-, 1,1,1-Tribrom-, 1,1,2-Tribrom-, 1,1,1,2-Tetrachlor-, 1,1,2,2-Tetrachlor-, 1,1,1,2-Tetrabrom- und 1,1,2,2-Tetrabromethan und vor allem Dichlormethan (Methylenchlorid) und Trichlormethan (Chloroform).

Die Menge an Lösungsmittel ist an sich nicht kritisch. In der Regel werden 30 bis 200 kg, bevorzugt 100 bis 150 kg, Lösungsmittel je kg Perylimid II eingesetzt.

Die Reaktionstemperaturen liegen generell bei 40 bis 140°C (insbesondere bis 90°C). Je nach dem gewünschten Substitutionsgrad werden zweckmäßigerweise niedrigere oder höhere Temperaturen aus dem genannten Bereich ausgewählt.

Soll lediglich in der 9-Position des Perylimids halogeniert werden, so sind Temperaturen von 40 bis 50°C vorzuziehen, wobei sich die Verwendung von Methylenchlorid als Lösungsmittel empfiehlt.

Sollen weitere Positionen des Perylengerüsts halogeniert werden, so sind Temperaturen von 60 bis 90°C bevorzugt. Die Trihalogenierung findet vorzugsweise bei 60 bis 70°C statt, die Pentachlorierung im allgemeinen bei etwa 75°C, wobei sich Chloroform bzw. 1,1,2,2-Tetrachlormethan (oder auch Chlorbenzol) besonders als Lösungsmittel eignen.

Von besonderem Vorteil ist hier die selektive Halogenierung, insbesondere Bromierung, der oben genannten Positionen.

Das Molverhältnis von Halogen zu Perylimid II beträgt üblicherweise 10:1 bis 100:1, für die Bromierung vorzugsweise 40:1 bis 60:1 und für die Chlorierung bevorzugt 10:1 bis 40:1.

Die Halogenierung a) kann sowohl bei Normaldruck als auch bei einem Überdruck bis zu etwa 10 bar durchgeführt werden und ist in der Regel nach 2 bis 10 h, besonders 5 bis 7 h, beendet. Die Reaktionszeiten hängen selbstverständlich auch vom gewünschten Halogenierungsgrad ab und nehmen mit zunehmendem Halogengehalt ebenfalls zu.

In Schritt, a) geht man verfahrenstechnisch zweckmäßigerweise wie folgt vor:

Man versetzt eine Lösung des Perylimids II im inerten Lösungsmittel, gegebenenfalls unter Druck, mit Halogen und erhitzt unter starkem Rühren auf die Reaktionstemperatur. Nach 2 bis 10 h Reaktionszeit, Abkühlen und gegebenenfalls Entspannen fällt man das Reaktionsgemisch auf Wasser, entfernt den Halogenüberschuß z.B. durch Versetzen mit alkalischer Sulfitlösung, trennt die das Halogenierungsprodukt enthaltende Phase ab und entfernt das Lösungsmittel.

Die in Schritt a) erhaltenen Halogenperylimide der Formel III 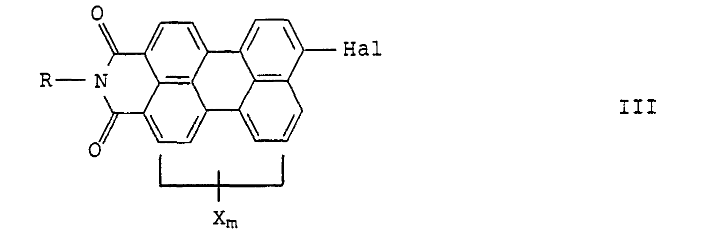 in der Hal Chlor oder Brom und m 0 bis 4 bedeutet, (bzw. die entsprechenden Halogenperylimide IIIa - IIId) können ohne weitere Reinigung direkt für den nächsten Reaktionsschritt eingesetzt werden.

Dieser Schritt (b) besteht in den Fällen der Quaterrylimide Ia, Ib und Id darin, jeweils zwei der entsprechenden Halogenperylimidmoleküle III zu einem Biperylenderivatmolekül der Formel IV 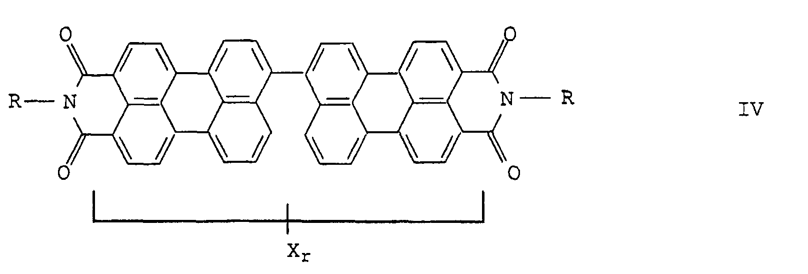 in der r 0 bis 8 bedeutet (bzw. dem entsprechenden Biperylenderivatmolekül IVa, IVb oder IVd) zu kuppeln.

Im Fall der Quaterrylimide Ic wird zuerst noch ein Zwischenschritt a') eingeschoben, bei dem die im Halogenperylimidmolekül IIIb enthaltenen Halogenatome mit Ausnahme des Halogenatoms in der 9-Position durch Umsetzung mit einem Nucleophil der Formel

Z―K

in der Z Aryloxy, Arylthio, Hetaryloxy oder Hetarylthio, das jeweils durch C₁-C₄-Alkyl oder C₁-C₄-Alkoxy substituiert sein kann, und K ein Alkalimetallkation oder Wasserstoff bedeutet, in Gegenwart einer tertiären stickstoffbasischen Verbindung als Lösungsmittel und gegebenenfalls einer anorganischen Base durch Reste Z ausgetauscht werden, wobei die Halogenperylimide IIIc erhalten werden, die anschließend ebenfalls gekuppelt werden.

Als tertiäre stickstoffbasische Lösungsmittel eignen sich beispielsweise aromatische Heterocyclen, wie Chinolin, Isochinolin und Chinaldin, und insbesondere cyclische Amide, wie vor allem N-Methylpyrrolidon.

Die Menge an Lösungsmittel ist an sich nicht kritisch und beträgt im allgemeinen 10 bis 100 kg, vorzugsweise 20 bis 40 kg, je kg IIIb.

Geeignete anorganische Basen sind z.B. Alkalimetallcarbonate und -hydroxide, wie insbesondere Kalium- und Natriumcarbonat und Kalium- und Natriumhydroxid, die bevorzugt als Feststoff eingesetzt werden.

Bei Verwendung von freien Alkoholen oder Thiolen Z-H als Nucleophilen wird in der Regel die 2- bis 5fache, bevorzugt die 2- bis 3fache, molare Menge anorganische Base, bezogen auf IIIb, benötigt. Werden direkt die Alkalimetallalkoholate oder -thiolate eingesetzt, kann auf den Zusatz der anorganischen Base verzichtet werden.

Es empfiehlt sich, pro mol auszutauschendes Halogenatom ein mol Nucleophil einzusetzen. Von besonderer Bedeutung ist, daß das Halogenatom in der 9-Position des Perylimids nicht substituiert wird, was nicht zu erwarten war.

Die Reaktionstemperatur beträgt beim Substitutionsschritt a') im allgemeinen 90 bis 160°C, vorzugsweise 110 bis 130°C.

Man kann unter Schutzgas (z.B. Stickstoff) arbeiten.

Gängige Reaktionszeiten liegen bei etwa 2 bis 10 h, insbesondere 5 bis 7 h.

Gewünschtenfalls kann man das Verfahren cyclisch führen, d.h. das in Schritt a') als Nebenprodukt entstehende enthalogenierte Substitutionsprodukt erneut gemäß Schritt a) halogenieren (bevorzugt bromieren) und dann gemäß Schritt b) kuppeln.

In Schritt a') geht man verfahrenstechnisch zweckmäßigerweise wie folgt vor:

Man legt Halogenperylimid IIIb, Nucleophil und gegebenenfalls feste anorganische Base im Lösungsmittel vor und erhitzt, gewünschtenfalls unter Schutzgas, etwa 2 bis 10 h auf die Reaktionstemperatur. Nach Abkühlen trägt man die Reaktionsmischung unter Rühren in verdünnte anorganische Säure, z.B. verdünnte Salzsäure, ein und filtriert den gebildeten Niederschlag ab, den man anschließend mit Wasser wäscht, trocknet und gegebenenfalls an Kieselgel mit aromatischen Lösungsmitteln wie Toluol oder halogenierten Kohlenwasserstoffen wie Chloroform chromatographiert.

Das so erhaltene Halogenperylimid IIIc kann wie die weiteren Halogenperylimide IIIa, IIIb und IIId direkt für den Kupplungsschritt b), bei dem die Biperylenderivate IV gebildet werden, eingesetzt werden.

Den Kupplungsschritt b) kann man analog zur EP-A-596 292 in Gegenwart eines organischen Metallkomplexes als Katalysator, zusätzlicher freier Ligandmoleküle und eines inerten Verdünnungsmittels durchführen.

Geeignete inerte Verdünnungsmittel sind z.B. vor allem aliphatische Carbonsäureamide, wie bevorzugt N,N-Dimethylformamid und N,N-Dimethylacetamid, aliphatische und cycloaliphatische Ether, wie Dimethylether, 1,2-Dimethyloxyethan und Tetrahydrofuran, und Aromaten, wie Benzol, Toluol und Xylol.

Die Verdünnungsmittelmenge ist an sich nicht kritisch und beträgt in der Regel 20 bis 100 kg, vorzugsweise 25 bis 45 kg, je kg Halogenperylimid III.

Bei den als Katalysator dienenden organischen Metallkomplexen kommen neben Palladiumkomplexen wie Tetrakis(triphenylphosphin)palladium insbesondere Nickelkomplexe in Betracht, z.B. Bis (triphenylphosphin)nickel(II)chlorid, Tetrakis(triphenylphosphin)nickel, [1,2-Bis(diphenylphosphino)ethan]nickel(II)-chlorid und bevorzugt Bis(1,5-cyclooctadien) nickel.

Man kann die Katalysatoren auch in situ durch Zugabe von Metallsalzen oder -verbindungen, freien Liganden, wie Cyclooctadien, Bipyridyl, Triphenylphosphin, Trifluorphosphin, η-, δ- und π-gebundenen Olefinen, Cycloolefinen, Aromaten und Antiaromaten, Carbonylen, Wasserstoff und Halogen sowie auch deren Mischungen, und erforderlichenfalls Oxidations- oder Reduktionsmitteln erzeugen.

Im allgemeinen werden 40 bis 150 mol-%, vorzugsweise 50 bis 100 mol-%, organischer Metallkomplex, bezogen auf III, eingesetzt.

In der Regel empfiehlt sich stets die gleichzeitige Anwesenheit von freien Ligandmolekülen, wie insbesondere Mischungen von Cyclooctadien und Bipyridyl im molaren Verhältnis von 1:1 bis 8:1. Hierbei sind Mengen von üblicherweise 80 bis 900 mol-%, bevorzugt 80 bis 200 mol-%, bezogen auf III, geeignet.

Der Kupplungsschritt b) wird vorteilhaft bei einer Temperatur von 40 bis 80°C, vorzugsweise 60 bis 65°C, durchgeführt, wobei man auch unter Schutzgas (z.B. Argon) arbeiten kann.

Die Kupplungsreaktion ist in der Regel nach 36 bis 48 h beendet.

In Schritt b) geht man verfahrenstechnisch zweckmäßigerweise wie folgt vor:

Man legt Halogenperylimid III, metallorganischen Katalysator sowie freie Ligandmoleküle im inerten Verdünnungsmittel vor und erhitzt, gegebenenfalls unter Schutzgas, etwa 36 bis 48 h auf die Reaktionstemperatur. Nach Abkühlen trägt man das Reaktionsgemisch in Wasser, das gegebenenfalls Methanol enthalten kann, ein, gibt verdünnte anorganische Säure, z.B. verdünnte Salzsäure, zu und filtriert den gebildeten Niederschlag ab, den man anschließend mit Wasser wäscht, trocknet und gegebenenfalls an Kieselgel mit aromatischen Lösungsmitteln wie Toluol oder Xylol chromatographiert.

Die so erhaltenen Biperylenderivate IV werden dann in Schritt c) analog zur EP-A-596 292 durch Erhitzen in Gegenwart eines Oxidationsmittels und eines alkalischen Reaktionsmediums in die Quaterrylimide I überführt.

Geeignete Oxidationsmittel sind beispielsweise Aluminiumchlorid, Eisen(III)chlorid, Mangandioxid, Palladium(II)acetat, Vanadiumtrichloridoxid, Kupfer(II)chlorid, Natriumformaldehydsulfoxylat und vor allem Glucose.

In der Regel wird die 10- bis 50fache, vorzugsweise 20- bis 35fache, molare Menge an bevorzugtem Oxidationsmittel Glucose, bezogen auf IV, eingesetzt.

Als alkalisches Reaktionsmedium dient zweckmäßig eine Mischung von einem Alkalimetallhydroxid, wie besonders Natrium- und Kaliumhydroxid, und einem C₁-C₄-Alkanol, wie Methanol, Ethanol, Propanol, Isopropanol, Butanol, Isobutanol und sec.-Butanol.

Üblicherweise kommen 60 bis 250 kg, bevorzugt 100 bis 160 kg, Alkalimetallhydroxid und 40 bis 200 kg, vorzugsweise 70 bis 140 kg, Alkanol je kg IV zum Einsatz.

Die Oxidation c) wird vorteilhaft bei einer Temperatur von 60 bis 180°C, insbesondere 100 bis 130°C, durchgeführt, wobei man auch unter Schutzgas, z.B. Argon, arbeiten kann.

Die Oxidation ist in der Regel nach 1 bis 4 h, besonders 1 bis 2,5 h, beendet.

In Schritt c) geht man verfahrenstechnisch zweckmäßigerweise wie folgt vor:

Man legt Biperylenderivat IV, Alkalimetallhydroxid, Alkanol und Oxidationsmittel vor und erhitzt, gegebenenfalls unter Schutzgas, 1 bis 2,5 h auf die Reaktionstemperatur. Nach Abkühlen säuert man das Reaktionsgemisch mit wäßriger Mineralsäure, z.B. Salzsäure, an und filtriert den gebildeten Niederschlag ab, den man anschließend mit Wasser wäscht, trocknet und einer Extraktion mit inerten Lösungsmitteln, wie Chloroform, Diethylether, Methyl-tert.-butylether und Toluol, zur Abtrennung gegebenenfalls vorhandener Nebenprodukte unterzieht.

Im Fall der Quaterrylimide Id fallen in Schritt c) die unsubstituierten Quaterrylimide Id' an, die anschließend noch halogeniert werden (Schritt d)) sowie gewünschtenfalls einer Substitution der Halogenatome durch nucleophile Reste Z (Schritt e)) unterzogen werden können.

Der Halogenierungsschritt d) kann analog zum Halogenierungsschritt a), jedoch in der 1,5- bis 2fachen Lösungsmittelmenge und mit jeweils verdoppelten Reaktionszeiten, vorgenommen werden.

Entscheidend ist, daß bei der Halogenierung des Quaterrylenmoleküls Id' im Gegensatz zu Schritt a) die anti-Halogenierungsprodukte (z.B. 1,7,9,11,17,19-Hexabromquaterrylimid) entstehen.

Wie in Schritt a) erhöht sich der Halogenierungsgrad auch hier mit steigender Temperatur und zunehmender Reaktionszeit.

Beim Substitutionsschritt e) kann man ebenfalls analog zum entsprechenden Schritt a') vorgehen, auch hier sollte jedoch die 1,5- bis 2fache Lösungsmittelmenge verwendet und die Reaktionszeit verdoppelt werden.

Die erfindungsgemäßen Quaterrylimide I eignen sich vorteilhaft als Pigmente oder Fluoreszenzfarbstoffe. Sie können insbesondere zur Einfärbung von hochmolekularen organischen Materialien oder auch organisch/anorganischen Compositen verwendet werden.

Als Pigmente sind insbesondere Quaterrylimide der Formel I geeignet, in der R C₈-C₂₀-Alkyl, Phenyl, Pyridyl oder Pyrimidyl, das jeweils durch C₁-C₄-Alkyl ein- oder mehrfach oder durch Phenylazo oder Naphthylazo einfach substituiert sein kann, X Halogen und n 2 bis 8 bedeutet.

Als Fluoreszenzfarbstoffe eignen sich vor allem Quaterrylimide der Formel I, in der R C₅-C₈-Cycloalkyl oder Phenyl, Pyridyl oder Pyrimidyl, das jeweils durch C₁-C₄-Alkoxy, -CONHR² oder -NHCOR² (R²: C₁-C₄-Alkyl oder Phenyl, das durch C₁-C₄-Alkyl oder C₁-C₄-Alkoxy substituiert sein kann) ein- oder mehrfach substituiert ist, X Phenoxy, Phenylthio, Pyrimidyloxy oder Pyrimidylthio, das durch C₁-C₄-Alkyl substituiert sein kann, und n 2 bis 8 bedeutet.

### Beispiele

### A1) Herstellung von erfindungsgemäßen 1,6,9-Tribromperylen-3,4-dicarbonsäureimiden der Formel IIIa'

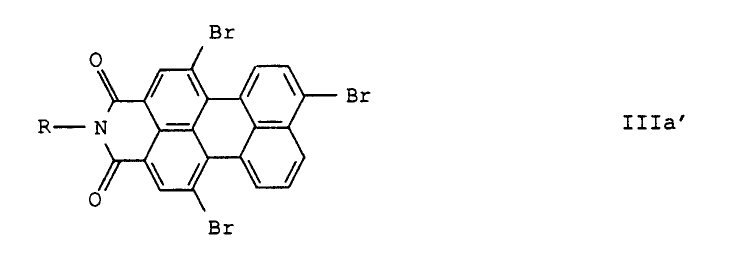

### Beispiel 1a

Eine Lösung von 12 g (25 mmol) N-(2,6-Diisopropylphenyl)perylen-3,4-dicarbonsäureimid in 1,5 l Chloroform wurde mit 75 ml (1,46 mol) Brom versetzt, unter starkem Rühren zum Rückfluß (etwa 61 °C) erhitzt und 6 h bei dieser Temperatur gehalten.

Nach Abkühlen auf Raumtemperatur wurde das Reaktionsgemisch unter Rühren in eine Lösung von 15 g Kaliumhydroxid und 10 g Natriumsulfit in 2 l Wasser eingetragen und solange portionsweise mit 6 g Kaliumhydroxid und 4 g Natriumsulfit versetzt, bis das überschüssige Brom vollständig entfernt war (erkennbar am Farbumschlag von dunkelrotbraun nach leuchtend orange). Die organische Phase wurde abgetrennt und das bromierte Perylimid IIIa' durch vollständiges Abziehen des Lösungsmittels isoliert.

Es wurden 19,5 g N-(2,6-Diisopropylphenyl)-1,6,9-tribromperylen-3,4-dicarbonsäureimid als orangefarbener Feststoff mit einem Wertgehalt von 84 % und einem Schmelzpunkt >300°C erhalten, was einer Ausbeute von 91 % entspricht.

### Beispiele 2a bis 5a

Auf analoge Weise zu Beispiel 1a wurden die in Tabelle A genannten Bromperylimide IIIa' durch Umsetzung der entsprechenden nichthalogenierten Perylimide mit Brom hergestellt. Der Übersichtlichkeit halber wird Beispiel la ebenfalls aufgeführt.

**Tabelle A**

| Bsp. | R | Ausbeute in % | Farbe | Schmp. [°C] |
|---|---|---|---|---|
| 1a | 2,6-Diisopropylphenyl | 91 | orange | >300 |
| 2a | Dodecyl | 92 | rot | >300 |
| 3a | 4-tert.-Butylphenyl | 100 | hellrot | >300 |
| 4a | 4-Phenylazophenyl | 95 | dunkelrot | >350 |
| 5a | 2-Methylphenyl | 98 | hellrot | >300 |

### A2) Herstellung von erfindungsgemäßen 1,7,9-Tribromperylen-3,4-dicarbonsäureimiden der Formel IIIa"

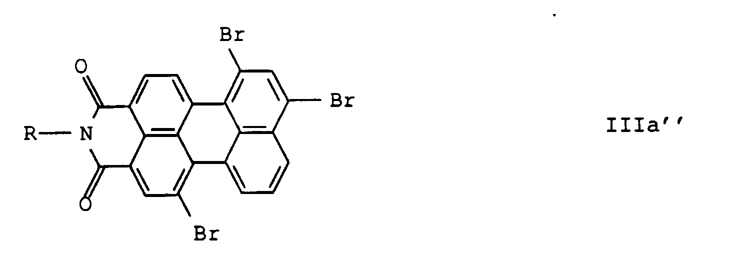

### Beispiel 6a (R = 2,6-Diisopropylphenyl)

15,9 g (25 mmol) N-(2,6-Diisopropylphenyl)-1,7-dibromperylen-3,4-dicarbonsäureimid wurden analog zu Beispiel la ebenfalls mit 75 ml (1,46 mol) Brom umgesetzt, jedoch bei 40°C in 1,2 l Methylenchlorid als Lösungsmittel.

Das als Edukt für die Herstellung des 1,7-dibromierten Perylimids dienende 1,7-Dibromperylen-3,4,9,10-tetracarbonsäuredianhydrid wurde durch selektive Bromierung von Perylen-3,4,9,10-tetracarbonsäuredianhydrid erhalten.

Dabei wurde eine Mischung von 292,5 g (0,75 mol) Perylen-3,4,9,10-tetracarbonsäuredianhydrid (Wertgehalt >98 %) und 4420 g 100 gew.-%iger Schwefelsäure nach 12stündigem Rühren und anschließender Zugabe von 7 g Iod auf 85°C erhitzt. Dann wurden 262,5 g (1,64 mol) Brom in 8 h zugetropft. Nach Abkühlen auf Raumtemperatur und Verdrängen des überschüssigen Broms durch Stickstoff wurde die Schwefelsäurekonzentration des Reaktionsgemisches durch portionsweise Zugabe von insgesamt 670 g Wasser in 1 h auf 86 Gew.-% erniedrigt. Nach dem Abkühlen des sich dabei auf 85°C erhitzenden Reaktionsgemisches auf Raumtemperatur wurde das ausgefallene Produkt über eine G4-Glasfritte abfiltriert, mit 3 kg 86 gew.-%iger Schwefelsäure gewaschen, dann in 5 1 Wasser aufgerührt, erneut abfiltriert, neutral gewaschen und bei 120°C im Vakuum getrocknet.

Bei der Umsetzung von Beispiel 6a wurden 18,5 g N-(2,6-Diisopropylphenyl)-1,7,9-tribromperylen-3,4-dicarbonsäureimid als orangeroter Feststoff mit einem Wertgehalt von 95 % und einem Schmelzpunkt >300°C erhalten, was einer Ausbeute von 98 % entspricht.

### B1) Herstellung von erfindungsgemäßen 1,6-Diaryloxy-9-bromperylen-3,4-dicarbonsäureimiden der Formel IIIb'

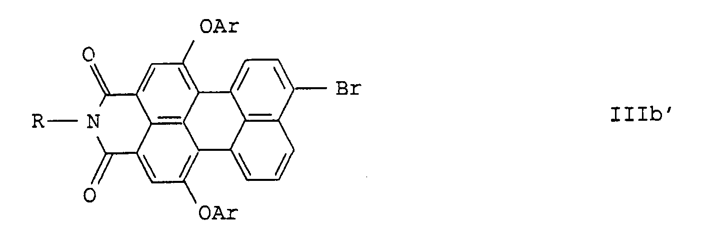

### Beispiel 1b

Eine Mischung von 16 g (22 mmol) des Tribromperylimids aus Beispiel 1a, 6,6 g (44 mmol) 4-tert.-Butylphenol und 6,9 g (50 mmol) Kaliumcarbonat in 500 ml N-Methylpyrrolidon wurde 6 h auf 120°C erhitzt.

Nach Abkühlen auf Raumtemperatur wurde das Reaktionsgemisch unter Rühren in 2 1 eines Gemisches von Wasser und 37 gew.-%iger Salzsäure im Gewichtsverhältnis 5:1 eingetragen. Der gebildete Niederschlag wurde abfiltriert, mit Wasser gewaschen, getrocknet und anschließend an Kieselgel (Säule 12 x 120 cm) mit Toluol chromatographiert, wobei das Produkt als erste, orangefarbene Fraktion eluiert wurde.

Nach Umkristallisation aus Methylenchlorid/Methanol wurden 6,3 g N-(2,6-Diisopropylphenyl)-1,6-di(4-tert.-butylphenoxy)-9-bromperylen-3,4-dicarbonsäureimid als roter Feststoff vom Schmelzpunkt 297°C erhalten, was einer Ausbeute von 33 % entspricht.

### Beispiele 1b' bis 3b'

Auf analoge Weise zu Beispiel 1b wurden die in Tabelle B genannten Perylimide IIIb' durch Umsetzung der entsprechenden Tribromperylimide IIIa' mit Phenolen hergestellt.

**Tabelle B**

| Bsp. | R | Ar | Ausbeute in % | Farbe | Schmp. [°C] |
|---|---|---|---|---|---|
| 1b | 2,6-Diisopropylphenyl | 4-tert.-Butylphenyl | 33 | rot | 297 |
| 1b' | 2,6-Diisopropylphenyl | Phenyl | 42 | rot | >300 |
| 2b | Dodecyl | 4-tert.-Butylphenyl | 33 | orange-rot | >300 |
| 3b | 4-tert.-Butylphenyl | 4-tert.-Butylphenyl | 16 | rot | >300 |
| 3b' | 4-tert.-Butylphenyl | Phenyl | 26 | rot | >300 |

### B2) Herstellung von erfindungsgemäßen 1,7-Diaryloxy-9-bromperylen-3,4-dicarbonsäureimiden der Formel IIIb"

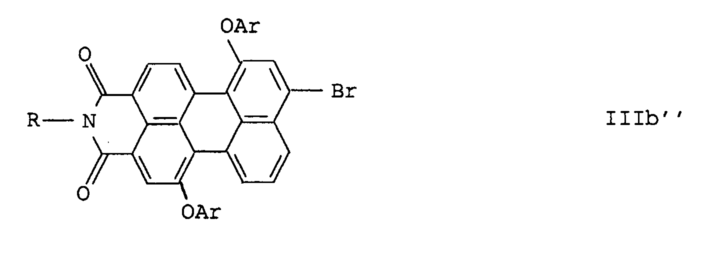

### Beispiel 6b (R = 2,6-Diisopropylphenyl; Ar = 4-tert.-Butylphenyl)

Analog zu Beispiel 1b wurde das Tribromperylimid aus Beispiel 6a mit 4-tert.-Butylphenol umgesetzt.

Es wurden 7,4 g N-(2,6-Diisopropylphenyl)-1,7-di(4-tert.-butylphenoxy)-9-bromperylen-3,4-dicarbonsäureimid als roter Feststoff vom Schmelzpunkt 289°C erhalten, was einer Ausbeute von 39 % entspricht.

### C1) Herstellung von 1,1',6,6'-Tetraaryloxy-3,3',4,4'-biperylentetracarbonsäurediimiden IV'

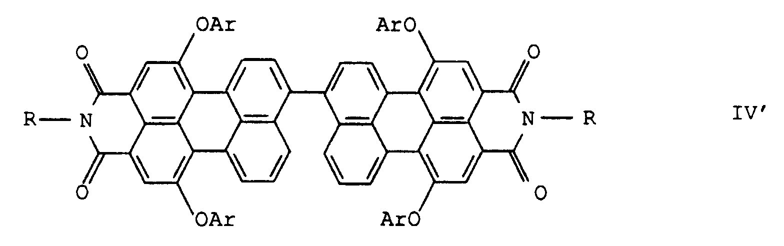

### Beispiel 1c

Eine Mischung von 385 mg (1,4 mmol) Bis(1,5-Cyclooctadien)nickel, 126 mg (1,17 mmol) 1,5-Cyclooctadien und 185 mg (1,17 mmol) 2,2'-Bipyridyl in 60 ml N,N-Dimethylformamid wurde in einem 100 ml Schlenkkolben unter Argon 1 h bei Raumtemperatur gerührt, mit 2 g (2,33 mmol) des Bromperylimids aus Beispiel 1b versetzt und anschließend 2 d bei 70°C gerührt.

Die Aufarbeitung wurde analog zu Beispiel 1b vorgenommen, jedoch wurde nur 1 l des Wasser/Salzsäure-Gemisches verwendet, und die Umkristallisation erfolgte aus Methylenchlorid.

Es wurden 1,55 g N,N'-Bis(2,6-diisopropylphenyl)-1,1',6,6'-tetra (4-tert.-butylphenoxy)-3,3',4,4'-biperylentetracarbonsäurediimid als rotes Pulver vom Schmelzpunkt 259°C erhalten, was einer Ausbeute von 86 % entspricht.

### Beispiele 1c' bis 3c'

Auf analoge Weise zu Beispiel 1c wurden die in Tabelle C genannten Biperylene IV' durch Kupplung der Bromperylimide IIIb' hergestellt.

**Tabelle C**

| Bsp. | R | Ar | Ausbeute in % | Farbe | Schmp. [°C] |
|---|---|---|---|---|---|
| 1c | 2,6-Diisopropylphenyl | 4-tert.-Butylphenyl | 86 | rot | 259 |
| 1c' | 2,6-Diisopropylphenyl | Phenyl | 88 | rot | >300 |
| 2c | Dodecyl | 4-tert.-Butylphenyl | 79 | rotbraun | >300 |
| 3c | 4-tert.-Butylphenyl | 4-tert.-Butylphenyl | 64 | rotbraun | >300 |
| 3c' | 4-tert.-Butylphenyl | Phenyl | 65 | rotbraun | >300 |

### C2) Herstellung von 1,1',7,7'-Tetraaroxy-3,3',4,4'-biperylentetracarbonsäureimiden IV''

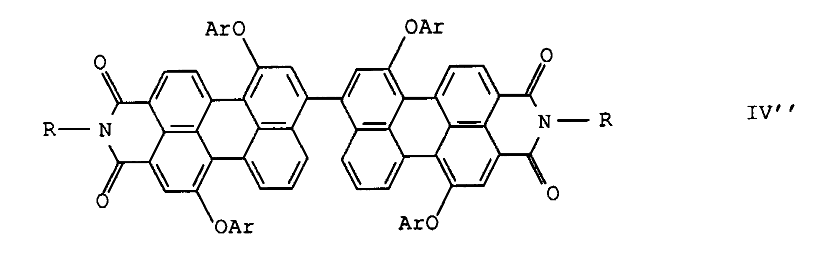

### Beispiel 6c (R = 2,6-Diisopropylphenyl; Ar = 4-tert.-Butylphenyl)

Analog zu Beispiel 1c wurde das Bromperylimid aus Beispiel 6b gekuppelt.

Es wurden 1,37 g N,N'-Bis(2,6-Diisopropylphenyl)-1,1',7,7'-tetra(4-tert.-butylphenoxy)-3,3'-4,4'-biperylentetracarbonsäurediimid als rotes Pulver vom Schmelzpunkt 273°C erhalten, was einer Ausbeute von 76 % entspricht.

### D1) Herstellung von erfindungsgemäßen 1,6,11,16-Tetraaryloxyquaterrylen-3,4:13,14-tetracarbonsäurediimiden I'

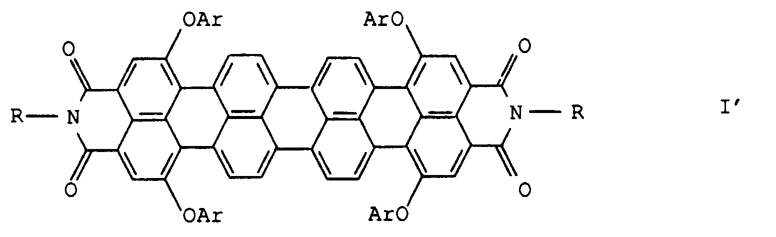

### Beispiel 1d

Eine Mischung von 80 g pulverisiertem Kaliumhydroxid, 80 ml Ethanol, 6 g Glucose und 500 mg (0,32 mmol) des Biperylenderivates aus Beispiel 1c wurde 2,5 h unter Argon auf 120°C erhitzt.

Nach Abkühlen auf Raumtemperatur wurde das Reaktionsgemisch in 420 ml Wasser eingetragen und mit 80 ml konzentrierter Salzsäure versetzt. Der entstandene Niederschlag wurde abfiltriert und nacheinander mit gesättigter wäßriger Kaliumcarbonatlösung, Wasser, Methanol und Diethylether gewaschen. Durch zusätzliche Extraktion mit Chloroform wurden die löslichen Nebenprodukte entfernt.

Es wurden 421 mg N,N'-Bis(2,6-diisopropylphenyl)-1,6,11,16-tetra(4-tert.-butylphenoxy)quaterrylen-3,4:13,14-tetracarbonsäurediimid als grünes Pulver vom Schmelzpunkt >300°C erhalten, was einer Ausbeute von 85 % entspricht.

Im folgenden werden einige analytische Daten für dieses Quaterrylimid angegeben:
¹H-NMR (500 MHz, CDCl₃):
   δ = 9,09 (d,4H); 8,32 (s,4H); 7,76 (d,4H); 7,41 (t,2H); 7,34 (d,8H): 7,28 (d,4H); 7,00 (d,8H); 2,76 (m,4H); 1,26 (s,36H); 1,13 (d,24H) ppm;
¹³C-NMR (125,5 MHz, CDCl₃):
   δ = 163,0; 153,6; 152,9; 147,0; 145,8; 131,2; 130,9; 130,8; 129,4; 129,2; 128,9; 127,7; 127,5; 127,2; 126,4; 125,2; 124,3; 123,9; 122,5; 121,6; 117,4; 34,4; 31,4; 29,2; 24,1 ppm;
IR (KBr) :
   ν=1707 (s,C=O), 1669 (s,C=O) cm⁻¹;
UV/VIS (CH₂Cl₂):
   λₘₐₓ (ε) = 262 (95819), 271 (97645), 382 (12895), 709 (71931), 781 (166571) nm;
Fluoreszenz (CH₂Cl₂):
   λₘₐₓ = 806 nm.

Von besonderer Bedeutung für die Anwendung als Fluoreszenzfarbstoff ist die Löslichkeit des Quaterrylimids in organischen Lösungsmitteln: 25-30 mg/ml Chloroform bei Raumtemperatur, 80 mg/ml Tetrachlorethan bei 135°C.

Im Vergleich dazu ist das aus der EP-A-596 292 bekannte unsubstituierte N,N'-Didodecylquaterrylimid in beiden Lösungsmitteln unlöslich und das analoge, unsubstituierte N,N'-Bis(2,6-diisopropylphenyl)quaterrylimid nahezu unlöslich (Löslichkeit <0,2 mg/ml Chloroform, 1-2 mg/ml Tetrachlorethan).

Von Interesse ist auch die bathochrome Verschiebung der langwelligsten Absorptionsbande im UV/VIS-Spektrum um 20 nm, verglichen mit dem entsprechenden unsubstituierten Quaterrylimid.

### D2) Herstellung von erfindungsgemäßen 1,7,10,16-Tetraaryloxyquaterrylen-3,4:13,14-tetracarbonsäurediimiden I"

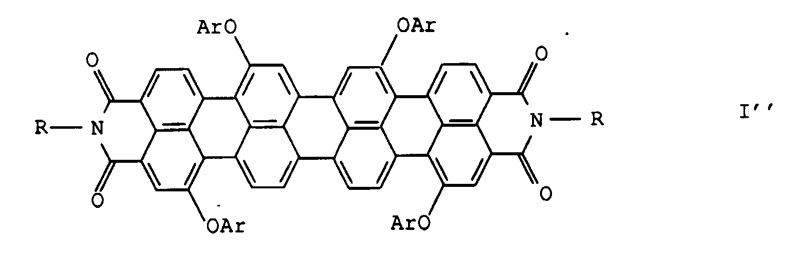

### Beispiel 6d (R = 2,6-Diisopropylphenyl; Ar = 4-tert.-Butylphenyl)

Analog zu Beispiel 1d wurde das Biperylen aus Beispiel 6c oxidiert.

Es wurden 318 mg N,N'-Bis(2,6-diisopropylphenyl)-1,7,10,16-tetra(4-tert.-butylphenoxy)quaterrylen-3,4:13,14-tetracarbonsäurediimid als blaugrünes Pulver vom Schmelzpunkt >300°C erhalten, was einer Ausbeute von 64 % entspricht.

### D3) Herstellung von N,N-Bis (2,6-diisopropylphenyl)-1,7,9,11,17,19-hexabromquaterrylen-3,4:13,14-tetracarbonsäurediimid I"'

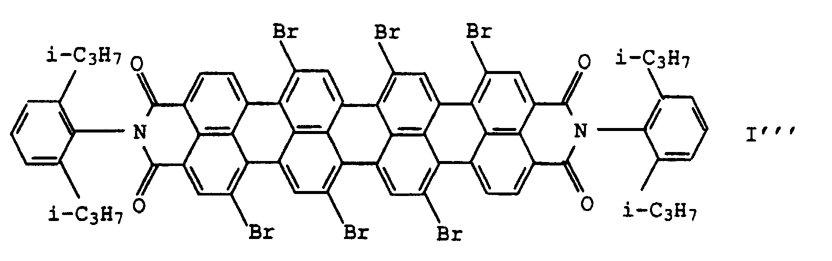

### Beispiel 7

1,0 g unsubstituiertes N,N'-Bis(2, 6-diisopropylphenyl)quaterrylimid, das durch Bromierung von unsubstituiertem N-2,6-Diisopropylperylen-3,4-dicarbonsäureimid analog zu Beispiel 6a zum 9-Bromperylimid, anschließende Kupplung analog zu Beispiel lc und Oxidation analog zu Beispiel ld erhalten wurde, wurde analog zu Beispiel la, jedoch unter Verdopplung der Reaktionszeit auf 12 h bromiert.

Es wurden 1,3 g I"' als dunkelroter Feststoff mit einem Wertgehalt von 85 % (15 % Tetrabromprodukt) und einem Schmelzpunkt >300°C erhalten, was einer Ausbeute von 91 % entspricht.

## Patentansprüche

1. Quaterrylentetracarbonsäurediimide der allgemeinen Formel I 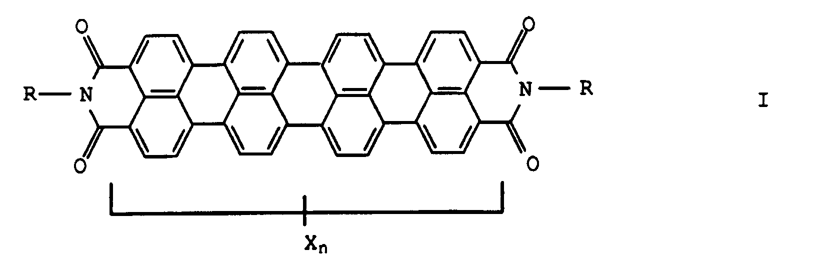 in der die Variablen folgende Bedeutung haben:
R Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano,
C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann, wobei
R¹ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR¹- unterbrochen sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Halogen substituiert sein kann, ein- oder mehrfach substituiert sein kann, wobei
R² Wasserstoff; C₁-C₁₈-Alkyl; Aryl oder Hetaryl, das jeweils durch C₁-C₆-Alkyl, C₁-C₆-Alkoxy, Halogen oder Cyano substituiert sein kann, bedeutet;
X Halogen; C₁-C₁₈-Alkyl; Aryloxy, Arylthio, Hetaryloxy oder Hetarylthio, das jeweils durch C₁-C₄-Alkyl oder C₁-C₄-Alkoxy substituiert sein kann;
n eine Zahl von 2 bis 12.

2. Quaterrylentetracarbonsäurediimide der Formel I nach Anspruch 1, in der die Variablen folgende Bedeutung haben:
R C₈-C₂₀-Alkyl, dessen Kohlenstoffkette durch eine oder mehrere Gruppierungen -O- oder -S- unterbrochen sein kann und das durch C₁-C₄-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann;
C₅-C₈-Cycloalkyl;
Phenyl oder Hetaryl, das jeweils durch C₁-C₁₂-Alkyl, C₁-C₄-Alkoxy, -CONHR² und/oder -NHCOR² ein- oder mehrfach und/oder durch Aryl- oder Hetarylazo, das jeweils durch C₁-C₁₀-Alkyl, C₁-C₆-Alkoxy, Halogen oder Cyano substituiert sein kann, einfach substituiert sein kann;
X Halogen; Phenoxy, Phenylthio, Pyridyloxy, Pyridylthio, Pyrimidyloxy oder Pyrimidylthio, das jeweils durch C₁-C₄-Alkyl oder C₁-C₄-Alkoxy substituiert sein kann;
n eine Zahl von 2 bis 8.

3. Quaterrylentetracarbonsäurediimide der Formel I nach Anspruch 1, in der die Variablen folgende Bedeutung haben:
R C₈-C₂₀-Alkyl, dessen Kohlenstoffkette durch eine oder mehrere Gruppierungen -O- unterbrochen sein kann und das durch C₁-C₄-Alkoxy substituiert sein kann;
C₅-C₈-Cycloalkyl;
Phenyl, Pyridyl oder Pyrimidyl, das jeweils durch C₁-C₄-Alkyl, C₁-C₄-Alkoxy, -CONHR² oder -NHCOR² ein- oder mehrfach und/oder durch Phenylazo oder Naphthylazo, das jeweils durch C₁-C₄-Alkyl, C₁-C₄-Alkoxy oder Cyano substituiert sein kann, einfach substituiert sein kann, wobei
R² C₁-C₄-Alkyl oder Phenyl, das durch C₁-C₄-Alkyl oder C₁-C₄-Alkoxy substituiert sein kann, bedeutet;
X Halogen; Phenoxy, Phenylthio, Pyrimidyloxy oder Pyrimidylthio, das jeweils durch C₁-C₄-Alkyl substituiert sein kann;
n eine Zahl von 2 bis 8.

4. Verfahren zur Herstellung von Quaterrylentetracarbonsäurediimiden der Formel Ia 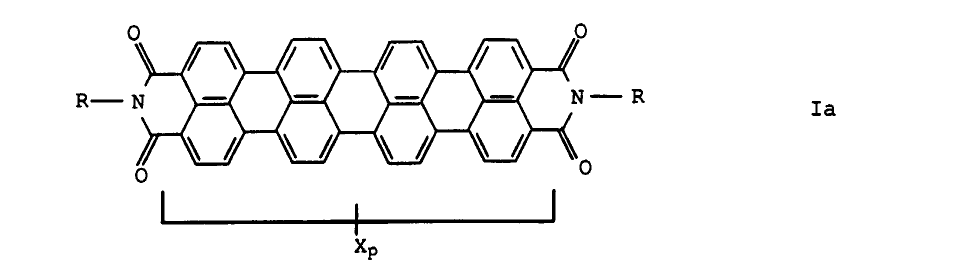 in der die Variablen folgende Bedeutung haben:
R Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano,
C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann, wobei
R¹ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR¹- unterbrochen sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Halogen substituiert sein kann, ein- oder mehrfach substituiert sein kann, wobei
R² Wasserstoff: C₁-C₁₈-Alkyl; Aryl oder Hetaryl, das jeweils durch C₁-C₆-Alkyl, C₁-C₆-Alkoxy, Halogen oder Cyano substituiert sein kann, bedeutet;
X Halogen; C₁-C₁₈-Alkyl; Aryloxy, Arylthio, Hetaryloxy oder Hetarylthio, das jeweils durch C₁-C₄-Alkyl oder C₁-C₄-Alkoxy substituiert sein kann;
p eine Zahl von 4 bis 8,
dadurch gekennzeichnet, daß man
a) Perylen-3,4-dicarbonsäureimide der Formel IIa 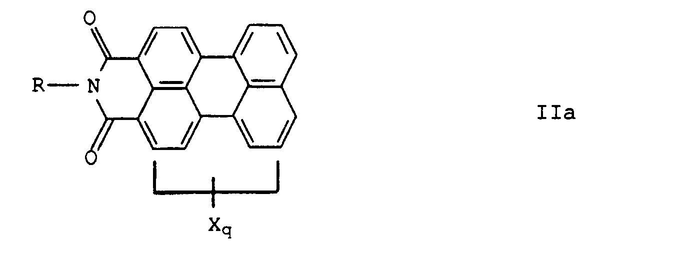 in der q 2 bis 4 bedeutet, mit elementarem Brom in Gegenwart eines inerten Verdünnungsmittels bei 40 bis 50°C zu bromierten Perylimiden der Formel IIIa 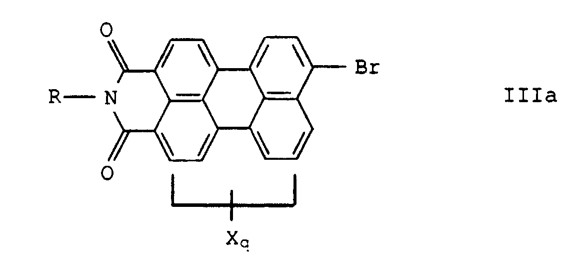 umsetzt,
b) die bromierten Perylimide IIIa in Gegenwart eines organischen Metallkomplexes als Katalysator, freier Ligandmoleküle und eines inerten Verdünnungsmittels zu Biperylenderivaten der Formel IVa 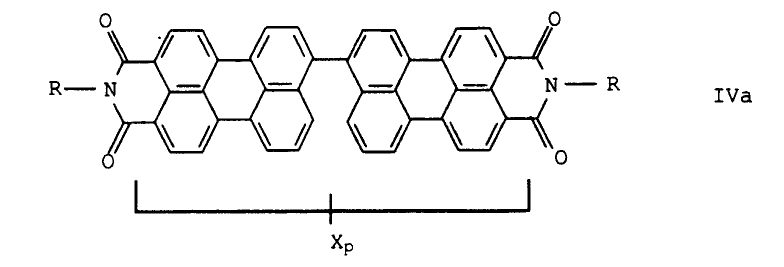 kuppelt und
c) die Biperylenderivate IVa durch Erhitzen in Gegenwart eines Oxidationsmittels und eines alkalischen Reaktionsmediums in die Quaterrylentetracarbonsäurediimide Ia überführt.

5. Verfahren zur Herstellung von Quaterrylentetracarbonsäurediimiden der Formel Ib 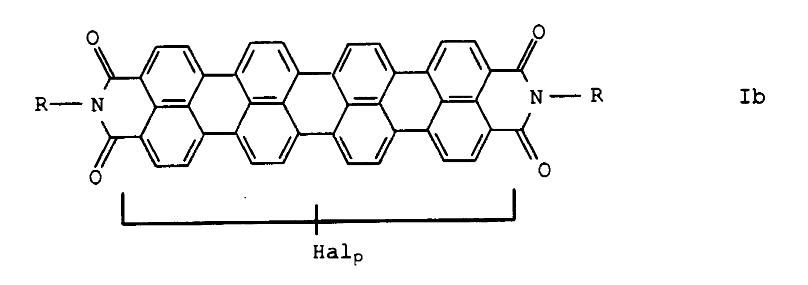 in der die Variablen folgende Bedeutung haben:
R Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann, wobei
R¹ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR¹- unterbrochen sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Halogen substituiert sein kann, ein- oder mehrfach substituiert sein kann, wobei
R² Wasserstoff; C₁-C₁₈-Alkyl; Aryl oder Hetaryl, das jeweils durch C₁-C₆-Alkyl, C₁-C₆-Alkoxy, Halogen oder Cyano substituiert sein kann, bedeutet,
Hal Chlor oder Brom;
p eine Zahl von 4 bis 8,
dadurch gekennzeichnet, daß man
a) Perylen-3,4-dicarbonsäureimide der Formel IIb 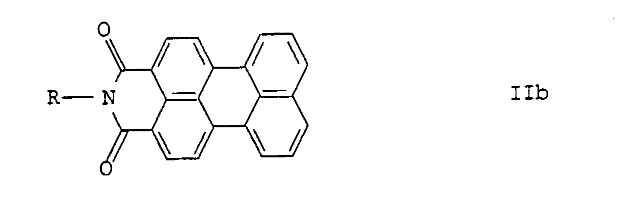 mit elementarem Halogen in Gegenwart eines inerten Verdünnungsmittels bei 60 bis 140°C zu halogenierten Perylimiden der Formel IIIb 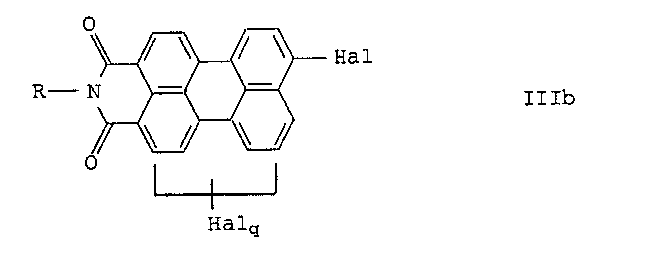 in der Hal Brom oder Chlor und q 2 bis 4 bedeutet, umsetzt,
b) die halogenierten Perylimide IIIb in Gegenwart eines organischen Metallkomplexes als Katalysator, freier Ligandmoleküle und eines inerten Verdünnungsmittels zu Biperylenderivaten der Formel IVb 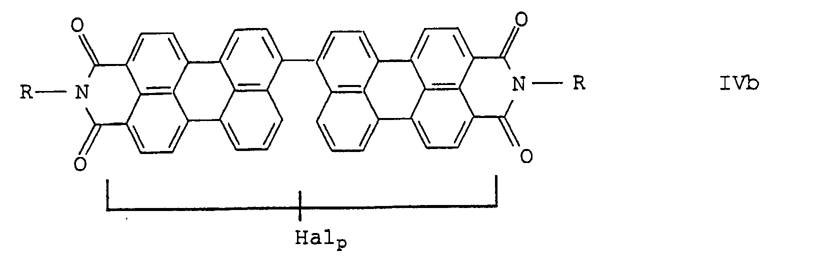 kuppelt und
c) die Biperylenderivate IVb durch Erhitzen in Gegenwart eines Oxidationsmittels und eines alkalischen Reaktionsmediums in die Quaterrylentetracarbonsäurediimide Ib überführt.

6. Verfahren zur Herstellung von Quaterrylentetracarbonsäurediimiden der Formel Ic 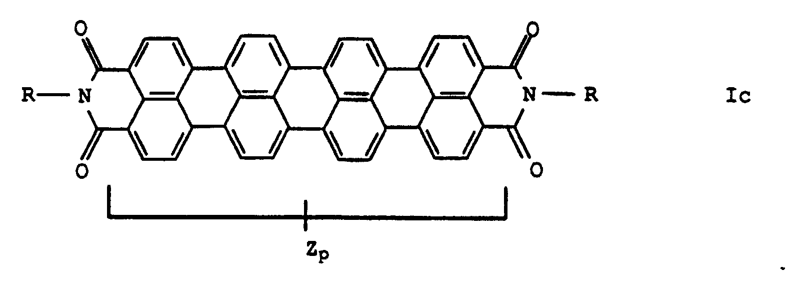 in der die Variablen folgende Bedeutung haben:
R Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano,
C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann, wobei
R¹ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR¹- unterbrochen sein kann;
Aryl oder Hetaryl, das jeweils durch C₁-C₁₈-Alkyl, C₁-C₆-Alkoxy, Cyano, -CONHR², -NBCOR² und/oder Aryl- oder Hetarylazo, das jeweils durch C₁-C₁₀-Alkyl, C₁-C₆-Alkoxy oder Halogen substituiert sein kann, ein- oder mehrfach substituiert sein kann, wobei
R² Wasserstoff; C₁-C₁₈-Alkyl; Aryl oder Betaryl, das jeweils durch C₁-C₆-Alkyl, C₁-C₆-Alkoxy, Halogen oder Cyano substituiert sein kann, bedeutet;
Z Aryloxy, Arylthio, Hetaryloxy oder Hetarylthio, das jeweils durch C₁-C₄-Alkyl oder C₁-C₄-Alkoxy substituiert sein kann;
p eine Zahl von 4 bis 8,
dadurch gekennzeichnet, daß man
a) Perylen-3,4-dicarbonsäureimide der Formel IIb 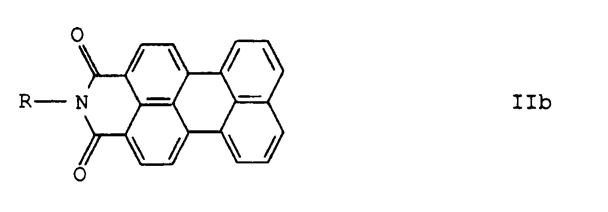 mit elementarem Halogen in Gegenwart eines inerten Verdünnungsmittels bei 60 bis 140°C zu halogenierten Perylimiden der Formel IIIb 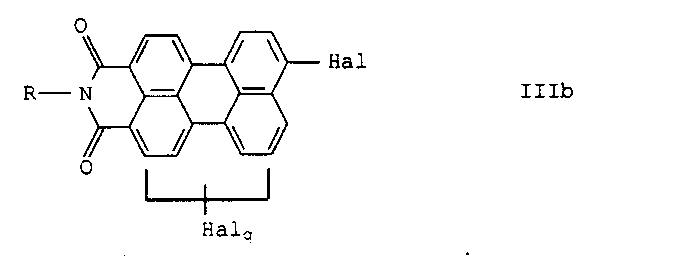 in der Hal Brom oder Chlor und q 2 bis 4 bedeutet, umsetzt,
a') die halogenierten Perylimide IIIb in Gegenwart einer tertiären stickstoffbasischen Verbindung als Lösungsmittel und gegebenenfalls einer anorganischen Base mit Nucleophilen der Formel
Z―K
in der K ein Alkalimetallkation oder Wasserstoff bedeutet, in substituierte Perylimide der Formel IIIc 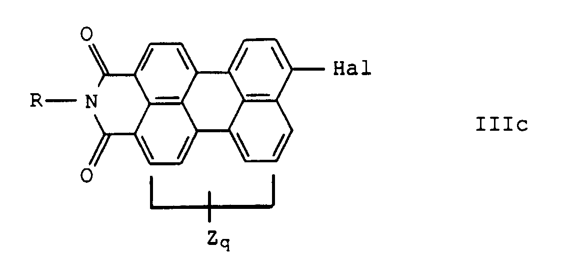 überführt,
b) die substituierten Perylimide IIIc in Gegenwart eines organischen Metallkomplexes als Katalysator, freier Ligandmoleküle und eines inerten Verdünnungsmittels zu Biperylenderivaten der Formel IVc 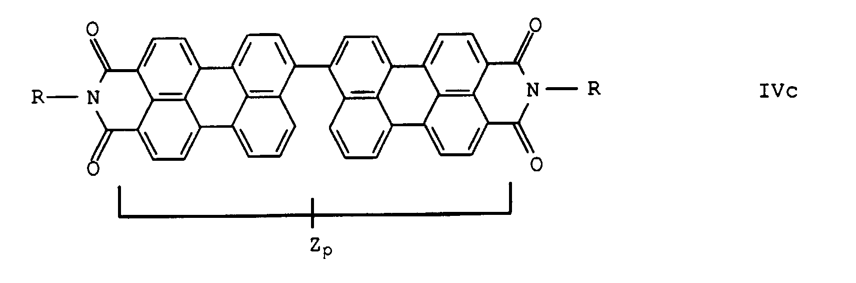 kuppelt und
c) die Biperylenderivate IVc durch Erhitzen in Gegenwart eines Oxidationsmittels und eines alkalischen Reaktionsmediums in die Quaterrylentetracarbonsäurediimide Ic überführt.

7. Verfahren zur Herstellung von Quaterrylentetracarbonsäurediimiden der Formel Id 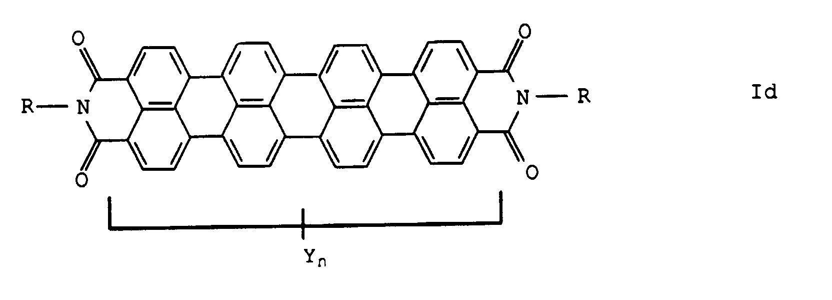 in der die Variablen folgende Bedeutung haben:
R Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann, wobei
R¹ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR¹- unterbrochen sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Halogen substituiert sein kann, ein- oder mehrfach substituiert sein kann, wobei
R² Wasserstoff; C₁-C₁₈-Alkyl; Aryl oder Hetaryl, das jeweils durch C₁-C₆-Alkyl, C₁-C₆-Alkoxy, Halogen oder Cyano substituiert sein kann, bedeutet;
Y Halogen; Aryloxy, Arylthio, Hetaryloxy oder Hetarylthio, das jeweils durch C₁-C₄-Alkyl oder C₁-C₄-Alkoxy substituiert sein kann;
n eine Zahl von 2 bis 12,
dadurch gekennzeichnet, daß man
a) Perylen-3,4-dicarbonsäureimide der Formel IIb 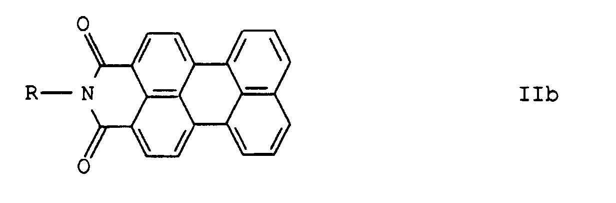 mit elementarem Brom in Gegenwart eines inerten Verdünnungsmittels bei 40 bis 50°C zu bromierten Perylimiden der Formel IIId 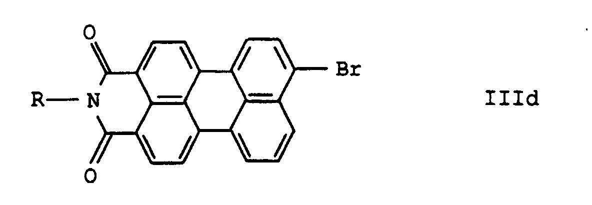 umsetzt,
b) die bromierten Perylimide IIId in Gegenwart eines organischen Metallkomplexes als Katalysator, freier Ligandmoleküle und eines inerten Verdünnungsmittels zu Biperylenderivaten der Formel IVd 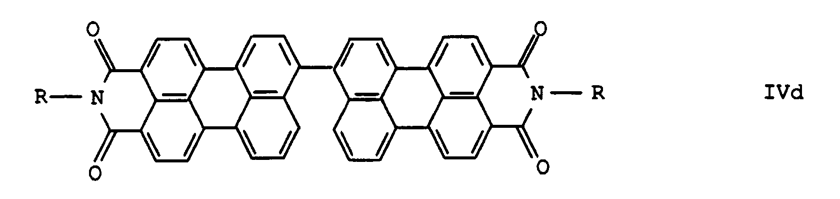 kuppelt,
c) die Biperylenderivate IVd durch Erhitzen in Gegenwart eines Oxidationsmittels und eines alkalischen Reaktionsmediums in unsubstituierte Quaterrylentetracarbonsäurediimide Id' 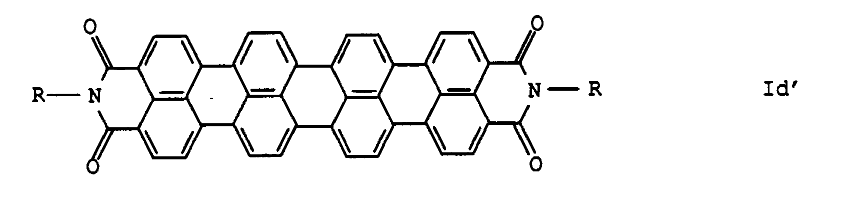 überführt,
d) die unsubstituierten Quaterrylentetracarbonsäurediimde Id' mit elementarem Halogen in Gegenwart eines inerten Verdünnungsmittels bei 60 bis 140°C zu den halogenierten Quaterrylentetracarbonsäurediimiden Id (Y : Hal) umsetzt und gewünschtenfalls
e) die halogenierten Quaterrylentetracarbonsäurediimide durch Umsetzung mit Nucleophilen der Formel
Z―K
in der Z Aryloxy, Arylthio, Hetaryloxy oder Hetarylthio, das jeweils durch C₁-C₄-Alkyl oder C₁-C₄-Alkoxy substituiert sein kann, und K ein Alkalimetallkation oder Wasserstoff bedeutet, in Gegenwart einer anorganischen Base und einer tertiären stickstoffbasischen Verbindung in die Quaterrylentetracarbonsäurediimide der Formel Id, in der Y für Z steht, überführt.

8. Verwendung von Quaterrylentetracarbonsäurediimiden der Formel I gemäß Anspruch 1 als Fluoreszenzfarbstoffe oder Pigmente.

9. 9-Halogenperylen-3,4-dicarbonsäureimide der allgemeinen For0 mel III 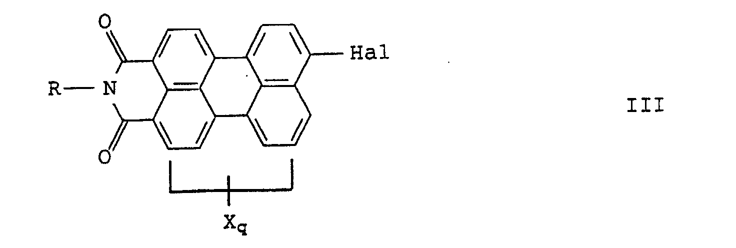 in der die Variablen folgende Bedeutung haben:
R Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy oder einen über ein Stickstoffatom gebundenen, 5- bis 7-gliedrigen heterocyclischen Rest, der weitere Heteroatome enthalten und aromatisch sein kann, ein- oder mehrfach substituiert sein kann, wobei
R¹ Wasserstoff oder C₁-C₆-Alkyl bedeutet;
C₅-C₈-Cycloalkyl, dessen Kohlenstoffgerüst durch eine oder mehrere Gruppierungen -O-, -S- und/oder -NR¹- unterbrochen sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy oder Halogen substituiert sein kann, ein- oder mehrfach substituiert sein kann, wobei
R² Wasserstoff; C₁-C₁₈-Alkyl; Aryl oder Hetaryl, das jeweils durch C₁-C₆-Alkyl, C₁-C₆-Alkoxy, Halogen oder Cyano substituiert sein kann, bedeutet;
X Halogen; C₁-C₁₈-Alkyl; Aryloxy, Arylthio, Hetaryloxy oder Hetarylthio, das jeweils durch C₁-C₄-Alkyl oder C₁-C₄-Alkoxy substituiert sein kann;
Hal Halogen;
q eine Zahl von 2 bis 4.

## Claims

1. Quaterrylenetetracarboxylic diimides of the general formula I 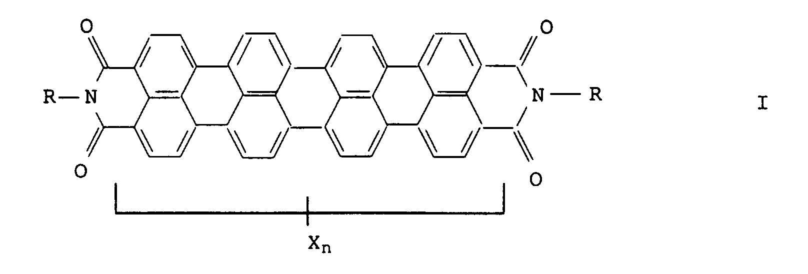 where
R is hydrogen;
C₁-C₃₀-alkyl whose carbon chain may be interrupted by one or more of -O-, -S-, -NR¹-, -CO- and/or -SO₂- and which may be monosubstituted or polysubstituted by cyano, C₁-C₆-alkoxy or a 5-, 6- or 7-membered heterocyclic radical which is attached via a nitrogen atom and which may contain further heteroatoms and may be aromatic, where
R¹ is hydrogen or C₁-C₆-alkyl;
C₅-C₈-cycloalkyl whose carbon skeleton may be interrupted by one or more of -O-, -S- and/or -NR¹-;
aryl or hetaryl, which may each be monosubstituted or polysubstituted by C₁-C₁₈-alkyl, C₁-C₆-alkoxy, cyano, -CONHR², -NHCOR² and/or aryl- or hetaryl-azo, which may each be substituted by C₁-C₁₀-alkyl, C₁-C₆-alkoxy or halogen, where
R² is hydrogen; C₁-C₁₈-alkyl; aryl or hetaryl, which may each be substituted by C₁-C₆-alkyl, C₁-C₆-alkoxy, halogen or cyano;
X is halogen; C₁-C₁₈-alkyl; aryloxy, arylthio, hetaryloxy or hetarylthio, which may each be substituted by C₁-C₄-alkyl or C₁-C₄-alkoxy;
n is from 2 to 12.

2. Quaterrylenetetracarboxylic diimides as claimed in claim 1 of the formula I where:
R is C₈-C₂₀-alkyl whose carbon chain may be interrupted by one or more of -O- or -S- and which may be monosubstituted or polysubstituted by C₁-C₄-alkoxy or a 5-, 6- or 7-membered heterocyclic radical which is attached via a nitrogen atom and which may contain further heteroatoms and may be aromatic;
C₅-C₈-cycloalkyl;
phenyl or hetaryl, which may each be monosubstituted or polysubstituted by C₁-C₁₂-alkyl, C₁-C₄-alkoxy, -CONHR² and/or -NHCOR² and/or monosubstituted by aryl- or hetaryl-azo, which may each be substituted by C₁-C₁₀-alkyl, C₁-C₆-alkoxy, halogen or cyano;
X is halogen; phenoxy, phenylthio, pyridyloxy, pyridylthio, pyrimidyloxy or pyrimidylthio, which may each be substituted by C₁-C₄-alkyl or C₁-C₄-alkoxy;
n is from 2 to 8.

3. Quaterrylenetetracarboxylic diimides as claimed in claim 1 of the formula I where:
R is C₈-C₂₀-alkyl whose carbon chain may be interrupted by one or more of -O- and which may be substituted by
C₁-C₄-alkoxy;
C₅-C₈-cycloalkyl;
phenyl, pyridyl or pyrimidyl, which may each be monosubstituted or polysubstituted by C₁-C₄-alkyl, C₁-C₄-alkoxy, -CONHR² or -NHCOR² and/or monosubstituted by phenylazo or naphthylazo, which may each be substituted by C₁-C₄-alkyl, C₁-C₄-alkoxy or cyano, where
R² is C₁-C₄-alkyl or phenyl which may be substituted by C₁-C₄-alkyl or C₁-C₄-alkoxy;
X is halogen; phenoxy, phenylthio, pyrimidyloxy or pyrimidylthio, which may each be substituted by C₁-C₄-alkyl;
n is from 2 to 8.

4. A process for preparing quaterrylenetetracarboxylic diimides of the formula Ia 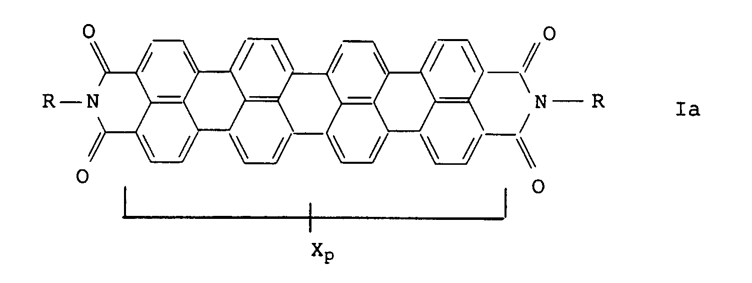 where:
R is hydrogen;
C₁-C₃₀-alkyl whose carbon chain may be interrupted by one or more of -O-, -S-, -NR¹-, -CO- and/or -SO₂- and which may be monosubstituted or polysubstituted by cyano,
C₁-C₆-alkoxy or a 5-, 6- or 7-membered heterocyclic radical which is attached via a nitrogen atom and which may contain further heteroatoms and may be aromatic, where
R¹ is hydrogen or C₁-C₆-alkyl;
C₅-C₈-cycloalkyl whose carbon skeleton may be interrupted by one or more of -O-, -S- and/or -NR¹-;
aryl or hetaryl, which may each be monosubstituted or polysubstituted by C₁-C₁₈-alkyl, C₁-C₆-alkoxy, cyano, -CONHR², -NHCOR² and/or aryl- or hetaryl-azo, which may each be substituted by C₁-C₁₀-alkyl, C₁-C₆-alkoxy or halogen, where
R² is hydrogen; C₁-C₁₈-alkyl; aryl or hetaryl, which may each be substituted by C₁-C₆-alkyl, C₁-C₆-alkoxy, halogen or cyano;
X is halogen; C₁-C₁₈-alkyl; aryloxy, arylthio, hetaryloxy or hetarylthio, which may each be substituted by C₁-C₄-alkyl or C₁-C₄-alkoxy;
p is from 4 to 8,
which comprises
a) reacting perylene-3,4-dicarbimides of the formula IIa 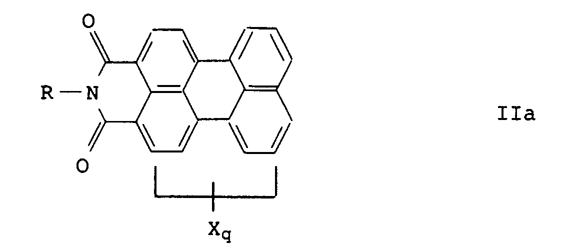 where q is from 2 to 4, with elemental bromine in the presence of an inert diluent at from 40 to 50°C to form brominated perylimides of the formula IIIa 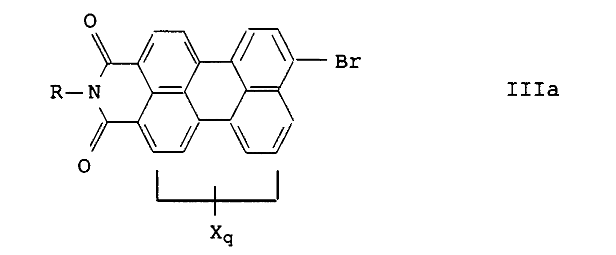
b) coupling the brominated perylimides IIIa in the presence of an organic metal complex as catalyst, free ligand molecules and an inert diluent to form biperylene derivatives of the formula IVa 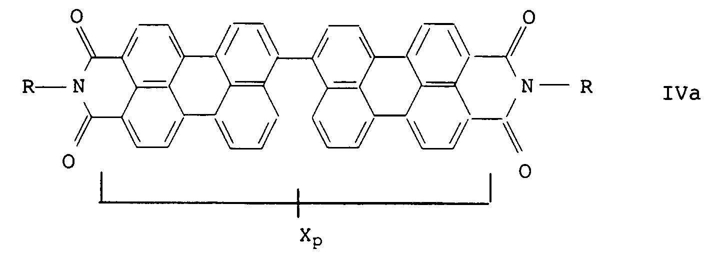 and
c) converting the biperylene derivatives IVa by heating in the presence of an oxidizing agent and of an alkaline reaction medium into the quaterrylenetetracarboxylic diimides Ia.

5. A process for preparing quaterrylenetetracarboxylic diimides of the formula Ib 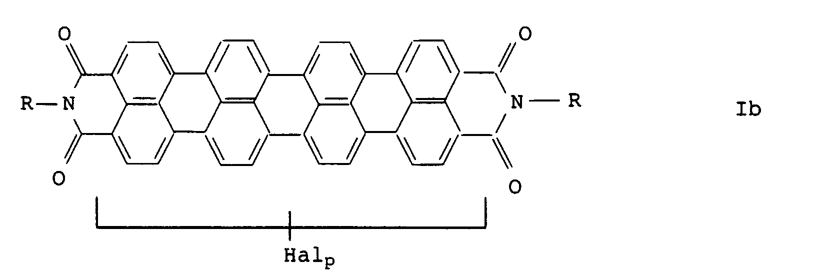 where:
R is hydrogen;
C₁-C₃₀-alkyl whose carbon chain may be interrupted by one or more of -O-, -S-, -NR¹-, -CO- and/or -SO₂- and which may be monosubstituted or polysubstituted by cyano, C₁-C₆-alkoxy or a 5-, 6- or 7-membered heterocyclic radical which is attached via a nitrogen atom and which may contain further heteroatoms and may be aromatic, where
R¹ is hydrogen or C₁-C₆-alkyl;
C₅-C₈-cycloalkyl whose carbon skeleton may be interrupted by one or more of -O-, -S- and/or -NR¹-;
aryl or hetaryl, which may each be monosubstituted or polysubstituted by C₁-C₁₈-alkyl, C₁-C₆-alkoxy, cyano, -CONHR², -NHCOR² and/or aryl- or hetaryl-azo, which may each be substituted by C₁-C₁₀-alkyl, C₁-C₆-alkoxy or halogen, where
R² is hydrogen; C₁-C₁₈-alkyl; aryl or hetaryl, which may each be substituted by C₁-C₆-alkyl, C₁-C₆-alkoxy, halogen or cyano;
Hal is chlorine or bromine;
p is from 4 to 8,
which comprises
a) reacting perylene-3,4-dicarbimides of the formula IIb 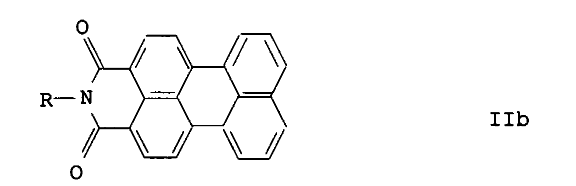 with elemental halogen in the presence of an inert diluent at from 60 to 140°C to form halogenated perylimides of the formula IIIb 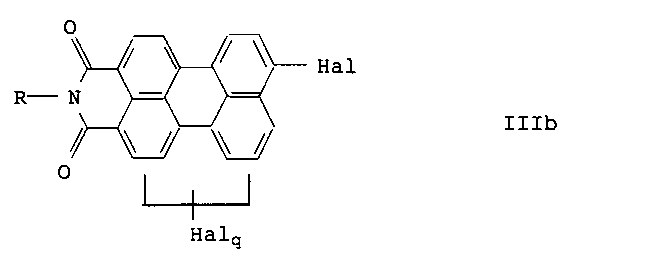 where Hal is bromine or chlorine and q is from 2 to 4,
b) coupling the halogenated perylimides IIIb in the presence of an organic metal complex as catalyst, free ligand molecules and an inert diluent to form biperylene derivatives of the formula IVb 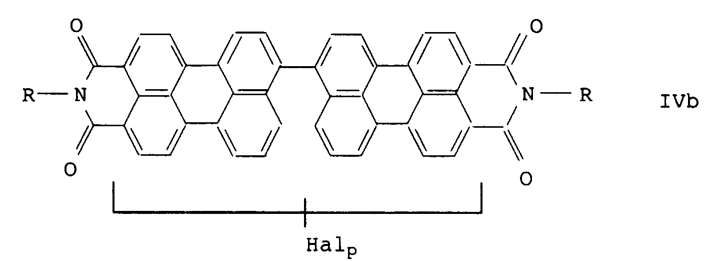 and
c) converting the biperylene derivatives IVb by heating in the presence of an oxidizing agent and of an alkaline reaction medium into the quaterrylenetetracarboxylic diimides Ib.

6. A process for preparing quaterrylenetetracarboxylic diimides of the formula Ic 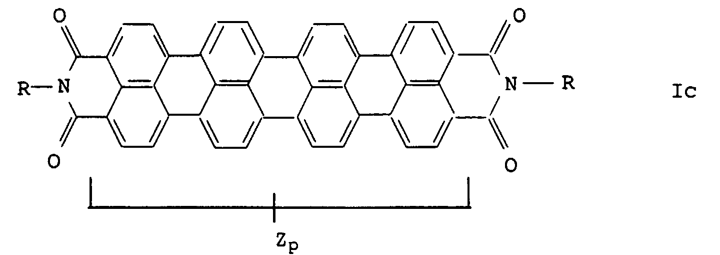 where:
R is hydrogen;
C₁-C₃₀-alkyl whose carbon chain may be interrupted by one or more of -O-, -S-, -NR¹-, -CO- and/or -SO₂- and which may be monosubstituted or polysubstituted by cyano, C₁-C₆-alkoxy or a 5-, 6- or 7-membered heterocyclic radical which is attached via a nitrogen atom and which may contain further heteroatoms and may be aromatic, where
R¹ is hydrogen or C₁-C₆-alkyl;
C₅-C₈-cycloalkyl whose carbon skeleton may be interrupted by one or more of -O-, -S- and/or -NR¹-;
aryl or hetaryl, which may each be monosubstituted or polysubstituted by C₁-C₁₈-alkyl, C₁-C₆-alkoxy, cyano, -CONHR², -NHCOR² and/or aryl- or hetaryl-azo, which may each be substituted by C₁-C₁₀-alkyl, C₁-C₆-alkoxy or halogen, where
R² is hydrogen; C₁-C₁₈-alkyl; aryl or hetaryl, which may each be substituted by C₁-C₆-alkyl, C₁-C₆-alkoxy, halogen or cyano;
Z is aryloxy, arylthio, hetaryloxy or hetarylthio, which may each be substituted by C₁-C₄-alkyl or C₁-C₄-alkoxy;
p is from 4 to 8,
which comprises
a) reacting perylene-3,4-dicarbimides of the formula IIb 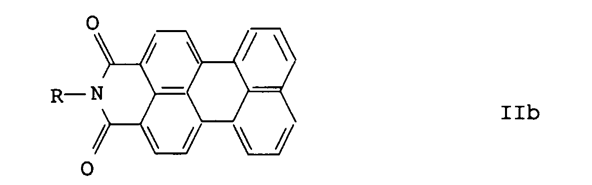 with elemental halogen in the presence of an inert diluent at from 60 to 140°C to form halogenated perylimides of the formula IIIb 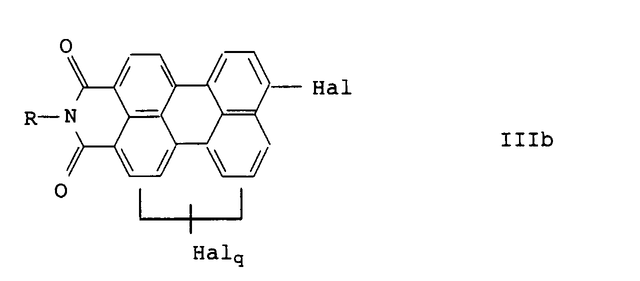 where Hal is bromine or chlorine and q is from 2 to 4,
a') converting the halogenated perylimides IIIb in the presence of a tertiary nitrogen base as solvent and optionally of an inorganic base with nucleophiles of the formula
Z-K
where K is an alkali metal cation or hydrogen, into substituted perylimides of the formula IIIc 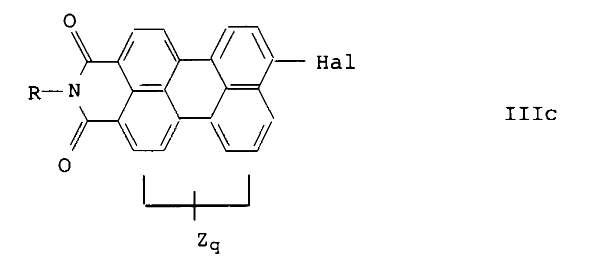
b) coupling the substituted perylimides IIIc in the presence of an organic metal complex as catalyst, free ligand molecules and an inert diluent to form biperylene derivatives of the formula IVc 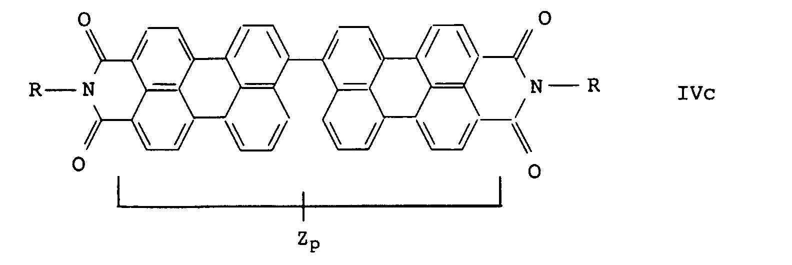 and
c) converting the biperylene derivatives IVc by heating in the presence of an oxidizing agent and of an alkaline reaction medium into the quaterrylenetetracarboxylic diimides Ic.

7. A process for preparing quaterrylenetetracarboxylic diimides of the formula Id 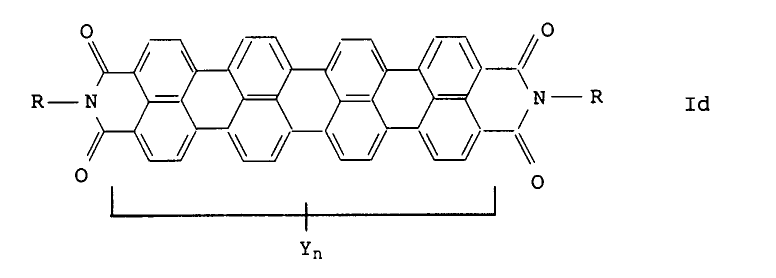 where:
R is hydrogen;
C₁-C₃₀-alkyl whose carbon chain may be interrupted by one or more of -O-, -S-, -NR¹-, -CO- and/or -SO₂- and which may be monosubstituted or polysubstituted by cyano, C₁-C₆-alkoxy or a 5-, 6- or 7-membered heterocyclic radical which is attached via a nitrogen atom and which may contain further heteroatoms and may be aromatic, where
R¹ is hydrogen or C₁-C₆-alkyl;
C₅-C₈-cycloalkyl whose carbon skeleton may be interrupted by one or more of -O-, -S- and/or -NR¹-;
aryl or hetaryl, which may each be monosubstituted or polysubstituted by C₁-C₁₈-alkyl, C₁-C₆-alkoxy, cyano, -CONHR², -NHCOR² and/or aryl- or hetaryl-azo, which may each be substituted by C₁-C₁₀-alkyl, C₁-C₆-alkoxy or halogen, where
R² is hydrogen; C₁-C₁₈-alkyl; aryl or hetaryl, which may each be substituted by C₁-C₆-alkyl, C₁-C₆-alkoxy, halogen or cyano;
Y is halogen; aryloxy, arylthio, hetaryloxy or hetarylthio, which may each be substituted by C₁-C₄-alkyl or C₁-C₄-alkoxy;
n is from 2 to 12,
which comprises
a) reacting perylene-3,4-dicarbimides of the formula IIb 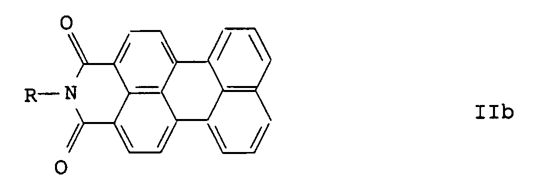 with elemental bromine in the presence of an inert diluent at from 40 to 50°C to form brominated perylimides of the formula IIId 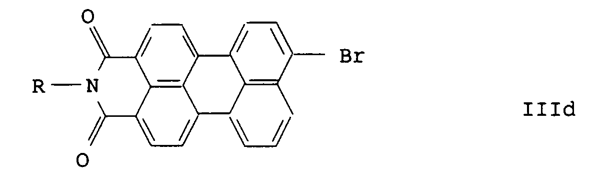
b) coupling the brominated perylimides IIId in the presence of an organic metal complex as catalyst, free ligand molecules and an inert diluent to form biperylene derivatives of the formula IVd 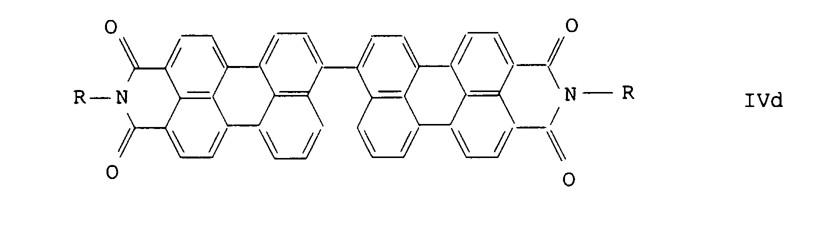
c) converting the biperylene derivatives IVd by heating in the presence of an oxidizing agent and of an alkaline reaction medium into unsubstituted quaterrylenetetracarboxylic diimides Id' 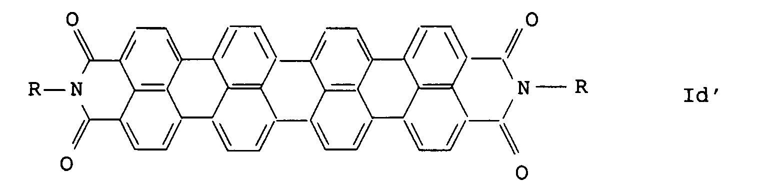
d) reacting the unsubstituted quaterrylenetetracarboxylic diimides Id' with elemental halogen in the presence of an inert diluent at from 60 to 140°C to form the halogenated quaterrylenetetracarboxylic diimides Id (Y : Hal), and if desired
e) converting the halogenated quaterrylenetetracarboxylic diimides by reaction with nucleophiles of the formula
Z-K
where Z is aryloxy, arylthio, hetaryloxy or hetarylthio, which may each be substituted by C₁-C₄-alkyl or C₁-C₄-alkoxy, and K is an alkali metal cation or hydrogen, in the presence of an inorganic base and of a tertiary nitrogen base into the quaterrylenetetracarboxylic diimide of the formula Id where Y is Z.

8. The use of quaterrylenetetracarboxylic diimides of the formula I as set forth in claim 1 as fluorescent dyes or pigments.

9. 9-Haloperylene-3,4-dicarbimides of the general formula III 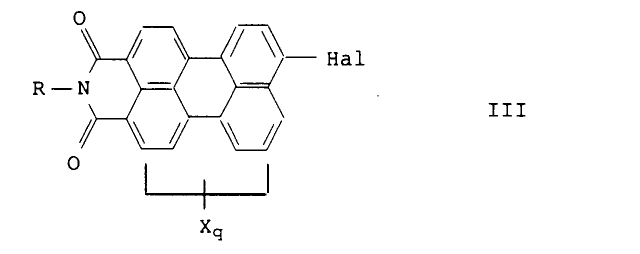 where:
R is hydrogen;
C₁-C₃₀-alkyl whose carbon chain may be interrupted by one or more of -O-, -S-, -NR¹-, -CO- and/or -SO₂- and which may be monosubstituted or polysubstituted by cyano, C₁-C₆-alkoxy or a 5-, 6- or 7-membered heterocyclic radical which is attached via a nitrogen atom and which may contain further heteroatoms and may be aromatic, where
R¹ is hydrogen or C₁-C₆-alkyl;
C₅-C₈-cycloalkyl whose carbon skeleton may be interrupted by one or more of -O-, -S- and/or -NR¹-;
aryl or hetaryl, which may each be monosubstituted or polysubstituted by C₁-C₁₈-alkyl, C₁-C₆-alkoxy, cyano, -CONHR², -NHCOR² and/or aryl- or hetaryl-azo, which may each be substituted by C₁-C₁₀-alkyl, C₁-C₆-alkoxy or halogen, where
R² is hydrogen; C₁-C₁₈-alkyl; aryl or hetaryl, which may each be substituted by C₁-C₆-alkyl, C₁-C₆-alkoxy, halogen or cyano;
X is halogen; C₁-C₁₈-alkyl; aryloxy, arylthio, hetaryloxy or hetarylthio, which may each be substituted by C₁-C₄-alkyl or C₁-C₄-alkoxy;
Hal is halogen;
q is from 2 to 4.

## Revendications

1. Quaterrylènetétracarboxodiimides de formule générale I 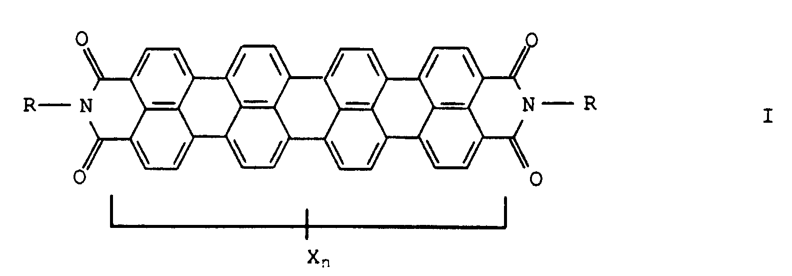 dans laquelle les variables ont les significations suivantes :
R est un atome d'hydrogène ;
un groupe alkyle en C₁-C₃₀, dont la chaîne carbonée peut être interrompue par un ou plusieurs groupements -O-, -S-, -NR¹-,-CO- et/ou -SO₂-, et qui peut être une ou plusieurs fois substitué par des groupes cyano, alcoxy en C₁-C₆, ou par un radical hétérocyclique ayant de 5 à 7 chaînons, lié par l'intermédiaire d'un atome d'azote, qui peut contenir d'autres hétéroatomes et peut être aromatique, où
R¹ est un atome d'hydrogène ou un groupe alkyle en C₁-C₆;
un groupe cycloalkyle en C₅-C₈, dont le squelette carboné peut être interrompu par un ou plusieurs groupements -O-, -S- et/ou-NR¹- ;
un groupe aryle ou hétéroaryle, dont chacun peut être une ou plusieurs fois substitué par des groupes alkyle en C₁-C₁₈, alcoxy en C₁-C₆, cyano, -CONHR², -NHCOR² et/ou aryl- ou hétéroarylazo, dont chacun peut être substitué par des groupes alkyle en C₁-C₁₀, alcoxy en C₁-C₆ ou halogéno, où
R² est un atome d'hydrogène, un groupe alkyle en C₁-C₁₈, aryle ou hétéroaryle dont chacun peut être substitué par des groupes alkyle en C₁-C₆, alcoxy en C₁-C₆, halogéno ou cyano ;
X est un atome d'halogène ; un groupe alkyle en C₁-C₁₈ ; aryloxy, arylthio, hétéroaryloxy ou hétéroarylthio, dont chacun peut être substitué par des groupes alkyle en C₁-C₄ ou alcoxy en C₁-C₄ ;
n est un nombre de 2 à 12.

2. Quaterrylènetétracarboxodiimides de formule I selon la revendication 1, dans laquelle les variables ont les significations suivantes :
R est un groupe alkyle en C₈-C₂₀, dont la chaîne carbonée peut être interrompue par un ou plusieurs groupements -O- ou -S-, et qui peut être une ou plusieurs fois substitué par des groupes alcoxy en C₁-C₄ ou par un radical hétérocyclique ayant de 5 à 7 chaînons, lié par l'intermédiaire d'un atome d'azote, et qui peut contenir d'autres hétéroatomes et qui peut être aromatique ;
un groupe cycloalkyle en C₅-C₈ ;
un groupe phényle ou hétéroaryle, dont chacun peut être une ou plusieurs fois substitué par des groupes alkyle en C₁-C₁₂, alcoxy en C₁-C₄, -CONHR² et/ou -NHCOR², et/ou peut être une fois substitué par des groupes aryl- ou hétéroarylazo, dont chacun peut être substitué par des groupes alkyle en C₁-C₁₀, alcoxy en C₁-C₆, halogéno ou cyano ;
X est un groupe halogéno ; phénoxy, phénylthio, pyridyloxy, pyridylthio, pyrimidyloxy ou pyrimidylthio, dont chacun peut être substitué par des groupes alkyle en C₁-C₄ ou alcoxy en C₁-C₄ ;
n est un nombre de 2 à 8.

3. Quaterrylènetétracarboxodiimides de formule I selon la revendication 1, dans laquelle les variables ont les significations suivantes :
R est un groupe alkyle en C₈-C₂₀ dont la chaîne carbonée peut être interrompue par un ou plusieurs groupements -O- et qui peut être substitué par des groupes alcoxy en C₁-C₄ ;
un groupe cycloalkyle en C₅-C₈;
un groupe phényle, pyridyle ou pyrimidyle, dont chacun peut être une ou plusieurs fois substitué par des groupes alkyle en C₁-C₄, alcoxy en C₁-C₄, -CONHR² ou -NHCOR² et/ou peut être une fois substitué par des groupes phénylazo ou naphtylazo, dont chacun peut être substitué par des groupes alkyle en C₁-C₄, alcoxy en C₁-C₄ ou cyano, où
R² est un groupe alkyle en C₁-C₄ ou phényle, qui peut être substitué par des groupes alkyle en C₁-C₄ ou alcoxy en C₁-C₄ ;
X est un groupe halogéno ; phénoxy, phénylthio, pyrimidyloxy ou pyrimidylthio, dont chacun peut être substitué par des groupes alkyle en C₁-C₄ ;
n est un nombre de 2 à 8.

4. Procédé de préparation de quaterrylènetétracarboxodiimides de formule générale Ia 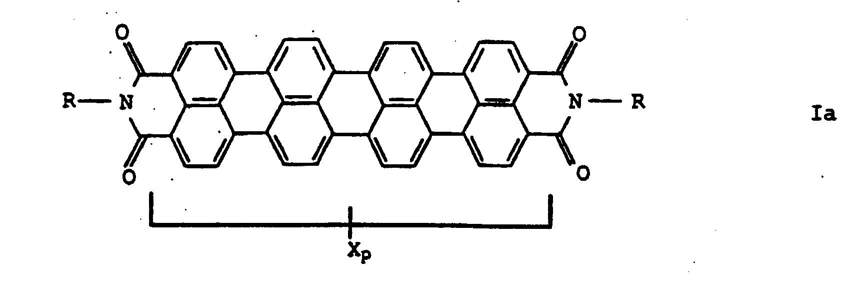 dans laquelle les variables ont les significations suivantes :
R est un atome d'hydrogène ;
un groupe alkyle en C₁-C₃₀, dont la chaîne carbonée peut être interrompue par un ou plusieurs groupements -O-, -S-, -NR¹-, -CO- et/ou -SO₂-, et qui peut être une ou plusieurs fois substitué par des groupes cyano, alcoxy en C₁-C₆, ou par un radical hétérocyclique ayant de 5 à 7 chaînons, lié par l'intermédiaire d'un atome d'azote, qui peut contenir d'autres hétéroatomes et peut être aromatique, où
R¹ est un atome d'hydrogène ou un groupe alkyle en C₁-C₆ ;
un groupe cycloalkyle en C₅-C₈, dont le squelette carboné peut être interrompu par un ou plusieurs groupements -O-, -S- et/ou -NR¹- ;
un groupe aryle ou hétéroaryle, dont chacun peut être une ou plusieurs fois substitué par des groupes alkyle en C₁-C₁₈, alcoxy en C₁-C₆, cyano, -CONHR², -NHCOR² et/ou aryl- ou hétéroarylazo, dont chacun peut être substitué par des groupes alkyle en C₁-C₁₀, alcoxy en C₁-C₆ ou halogéno, où
R² est un atome d'hydrogène, un groupe alkyle en C₁-C₁₈, aryle ou hétéroaryle dont chacun peut être substitué par des groupes alkyle en C₁-C₆, alcoxy en C₁-C₆, halogéno ou cyano ;
X est un atome d'halogène ; un groupe alkyle en C₁-C₁₈ ; aryloxy, arylthio, hétéroaryloxy ou hétéroarylthio, dont chacun peut être substitué par des groupes alkyle en C₁-C₄ ou alcoxy en C₁-C₄ ;
p est un nombre de 4 à 8,
caractérisé en ce que
a) on fait réagir des pérylène-3,4-dicarboximides de formule IIa 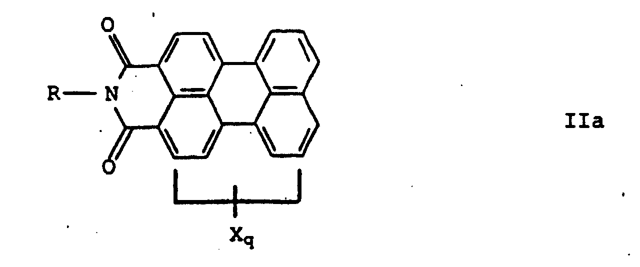 dans laquelle q vaut de 2 à 4, avec du brome élémentaire, en présence d'un diluant inerte à une température de 40 à 50°C, pour obtenir des pérylimides bromés de formule IIIa 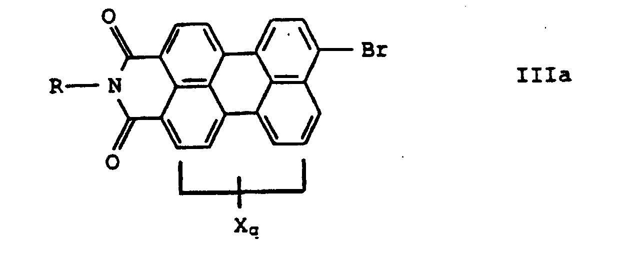
b) on couple les pérylimides bromés IIIa, en présence d'un complexe organométallique servant de catalyseur, de molécules de ligands libres et d'un diluant inerte, pour obtenir des dérivés du bipérylène de formule IVa 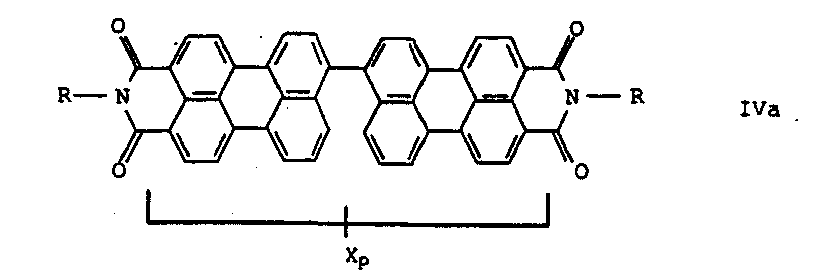 et
c) on convertit les dérivés du bipérylène IVa, par chauffage en présence d'un oxydant et d'un milieu de réaction alcalin, en les quaterrylènetétracarboxodiimides Ia.

5. Procédé de préparation de quaterrylènetétracarboxodiimides de formule générale Ib 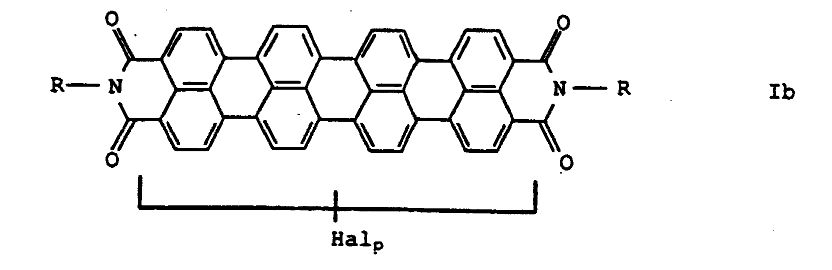 dans laquelle les variables ont les significations suivantes :
R est un atome d'hydrogène ;
un groupe alkyle en C₁-C₃₀, dont la chaîne carbonée peut être interrompue par un ou plusieurs groupements -O-, -S-, -NR¹-, -CO- et/ou -SO₂-, et qui peut être une ou plusieurs fois substitué par des groupes cyano, alcoxy en C₁-C₆, ou par un radical hétérocyclique ayant de 5 à 7 chaînons, lié par l'intermédiaire d'un atome d'azote, qui peut contenir d'autres hétéroatomes et peut être aromatique, où
R¹ est un atome d'hydrogène ou un groupe alkyle en C₁-C₆ ;
un groupe cycloalkyle en C₅-C₈, dont le squelette carboné peut être interrompu par un ou plusieurs groupements -O-, -S- et/ou -NR¹- ;
un groupe aryle ou hétéroaryle, dont chacun peut être une ou plusieurs fois substitué par des groupes alkyle en C₁-C₁₈, alcoxy en C₁-C₆, cyano, -CONHR², -NHCOR² et/ou aryl- ou hétéroarylazo, dont chacun peut être substitué par des groupes alkyle en C₁-C₁₀, alcoxy en C₁-C₆ ou halogéno, où
R² est un atome d'hydrogène, un groupe alkyle en C₁-C₁₈, aryle ou hétéroaryle dont chacun peut être substitué par des groupes alkyle en C₁-C₆, alcoxy en C₁-C₆, halogéno ou cyano ;
Hal est le chlore ou le brome ;
p est un nombre de 4 à 8,
caractérisé en ce que :
a) on fait réagir des pérylène-3,4-dicarboximides de formule IIb 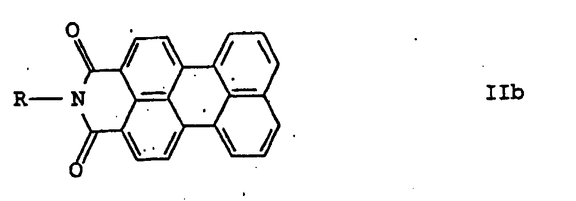 avec un halogène élémentaire en présence d'un diluant inerte à une température de 60 à 140°C, pour obtenir des pérylimides halogénés de formule IIIb 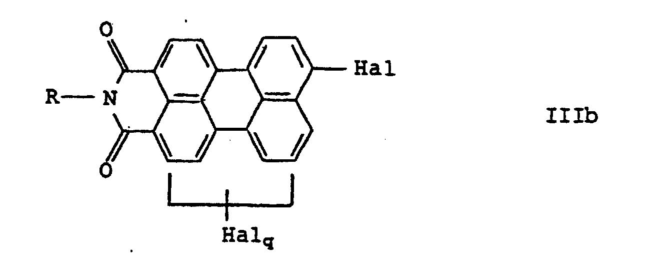 dans laquelle Hal est le brome ou le chlore et q vaut 2 à 4,
b) on couple les pérylimides halogénés IIIb en présence d'un complexe organométallique servant de catalyseur, d'une molécule de ligand libre et d'un diluant inerte, pour obtenir les dérivés du bipérylène de formule IVb 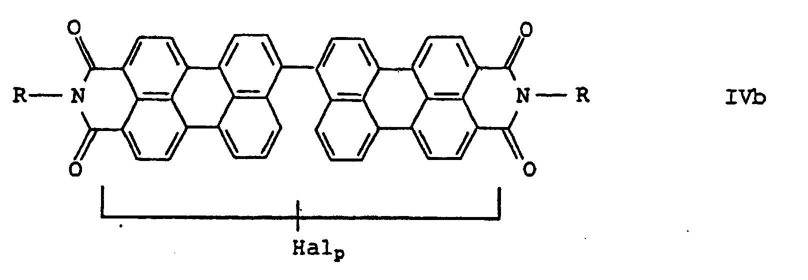
c) on convertit les dérivés du bipérylène IVb par chauffage en présence d'un oxydant et d'un milieu de réaction alcalin, en les quaterrylènetétracarboxodiimides Ib.

6. Procédé de préparation de quaterrylènetétracarboxodiimides de formule générale Ic 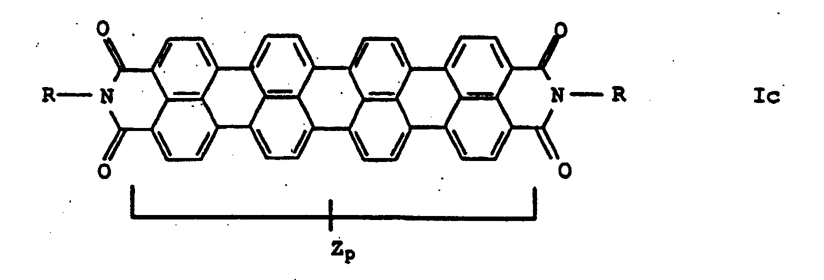 dans laquelle les variables ont les significations suivantes :
R est un atome d'hydrogène ;
un groupe alkyle en C₁-C₃₀, dont la chaîne carbonée peut être interrompue par un ou plusieurs groupements -O-, -S-, -NR¹-, -CO- et/ou -SO₂-, et qui peut être une ou plusieurs fois substitué par des groupes cyano, alcoxy en C₁-C₆, ou par un radical hétérocyclique ayant de 5 à 7 chaînons, lié par l'intermédiaire d'un atome d'azote, qui peut contenir d'autres hétéroatomes et peut être aromatique, où
R¹ est un atome d'hydrogène ou un groupe alkyle en C₁-C₆ ;
un groupe cycloalkyle en C₅-C₈, dont le squelette carboné peut être interrompu par un ou plusieurs groupements -O-, -S- et/ou -NR¹- ;
un groupe aryle ou hétéroaryle, dont chacun peut être une ou plusieurs fois substitué par des groupes alkyle en C₁-C₁₈, alcoxy en C₁-C₆, cyano, -CONHR², -NHCOR² et/ou aryl- ou hétéroarylazo, dont chacun peut être substitué par des groupes alkyle en C₁-C₁₀, alcoxy en C₁-C₆ ou halogéno, où
R² est un atome d'hydrogène, un groupe alkyle en C₁-C₁₈, aryle ou hétéroaryle dont chacun peut être substitué par des groupes alkyle en C₁-C₆, alcoxy en C₁-C₆, halogéno ou cyano ;
Z est un groupe aryloxy, arylthio, hétéroaryloxy ou hétéroarylthio, dont chacun peut être substitué par des groupes alkyle en C₁-C₄ ou alcoxy en C₁-C₄ ;
p est un nombre de 4 à 8,
caractérisé en ce que
a) on fait réagir des pérylène-3,4-dicarboximides de formule IIb 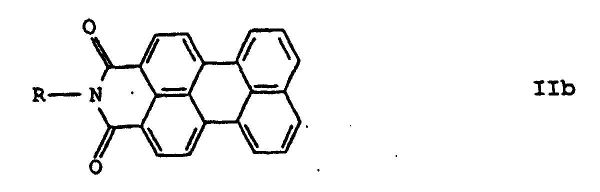 avec un halogène élémentaire en présence d'un diluant inerte à une température de 60 à 140°C, pour obtenir des pérylimides halogénés de formule IIIb 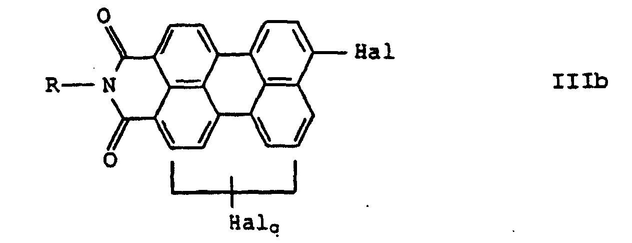 dans laquelle Hal est le brome ou le chlore et q vaut 2 à 4,
a') on convertit les pérylimides halogénés IIIb, en présence d'un composé basique à base d'azote tertiaire servant de solvant, et éventuellement d'une base minérale, avec des nucléophiles de formule
Z - K
dans laquelle K est le cation d'un métal alcalin ou un atome d'hydrogène, en des pérylimides substitués de formule IIIc 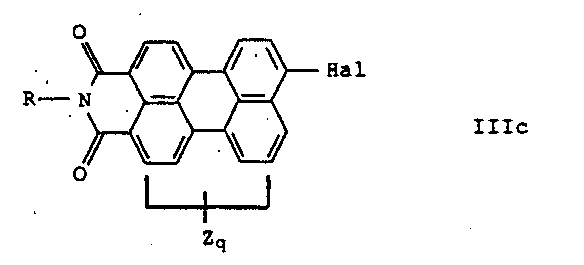
b) on couple les pérylimides substitués IIIc en présence d'un complexe organométallique servant de catalyseur, d'une molécule de ligand libre et d'un diluant inerte, en des dérivés du bipérylène de formule IVc 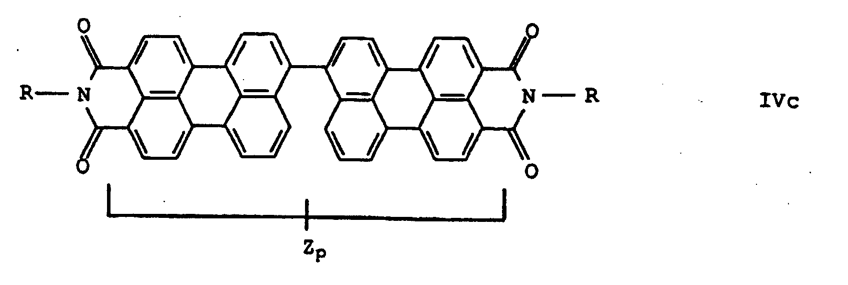 et
c) on convertit les dérivés du bipérylène IVc, par chauffage en présence d'un oxydant et d'un milieu de réaction alcalin, en les quaterrylènetétracarboxodiimides Ic.

7. Procédé de préparation de quaterrylènetétracarboxodiimides de formule générale Id 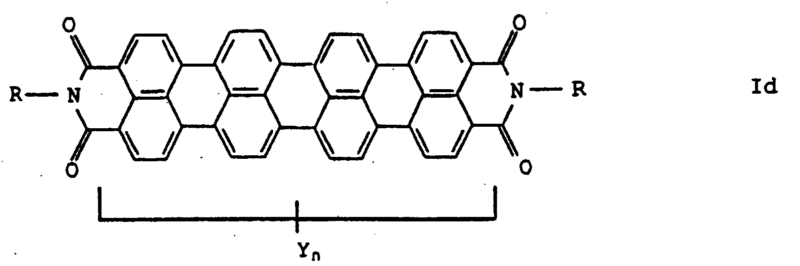 dans laquelle les variables ont les significations suivantes :
R est un atome d'hydrogène ;
un groupe alkyle en C₁-C₃₀, dont la chaîne carbonée peut être interrompue par un ou plusieurs groupements -O-, -S-, -NR¹-, -CO- et/ou -SO₂-, et qui peut être une ou plusieurs fois substitué par des groupes cyano, alcoxy en C₁-C₆, ou par un radical hétérocyclique ayant de 5 à 7 chaînons, lié par l'intermédiaire d'un atome d'azote, qui peut contenir d'autres hétéroatomes et peut être aromatique, où
R¹ est un atome d'hydrogène ou un groupe alkyle en C₁-C₆;
un groupe cycloalkyle en C₅-C₈, dont le squelette carboné peut être interrompu par un ou plusieurs groupements -O-, -S- et/ou -NR¹- ;
un groupe aryle ou hétéroaryle, dont chacun peut être une ou plusieurs fois substitué par des groupes alkyle en C₁-C₁₈, alcoxy en C₁-C₆, cyano, -CONHR², -NHCOR² et/ou aryl- ou hétéroarylazo, dont chacun peut être substitué par des groupes alkyle en C₁-C₁₀, alcoxy en C₁-C₆ ou halogéno, où
R² est un atome d'hydrogène, un groupe alkyle en C₁-C₁₈, aryle ou hétéroaryle dont chacun peut être substitué par des groupes alkyle en C₁-C₆, alcoxy en C₁-C₆, halogéno ou cyano ;
Y est un atome d'halogène ; un groupe aryloxy, arylthio, hétéroaryloxy ou hétéroarylthio, dont chacun peut être substitué par des groupes alkyle en C₁-C₄ ou alcoxy en C₁-C₄ ;
n est un nombre de 2 à 12,
caractérisé en ce que
a) on fait réagir des pérylène-3,4-dicarboximides de formule IIb 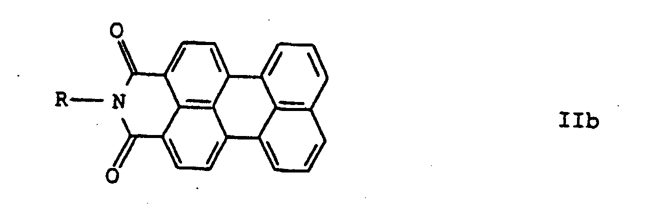 avec du brome élémentaire en présence d'un diluant inerte à une température de 40 à 50°C pour obtenir des pérylimides bromés de formule IIId 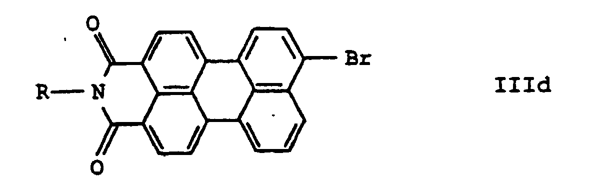
b) on couple les pérylimides bromés IIId en présence d'un complexe organométallique servant de catalyseur, d'une molécule de ligand libre et d'un diluant inerte, pour obtenir des dérivés du bipérylène de formule IVd 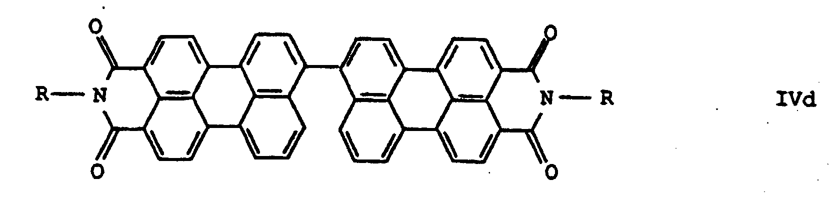
c) on convertit les dérivés du bipérylène IVd, par chauffage en présence d'un oxydant et d'un milieu de réaction alcalin, en les quaterrylènetétracarboxodiimides non-substitués Id' 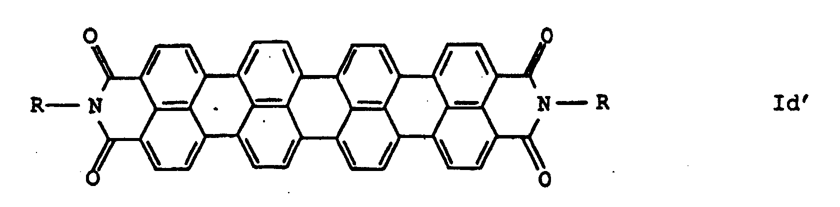
d) on fait réagir les quaterrylènetétracarboxodiimides non-substitués Id' avec de l'halogène élémentaire en présence d'un diluant inerte à une température de 60 à 140°C pour obtenir les quaterrylènetétracarboxodiimides halogénés Id (Y : Hal), et, si on le souhaite,
e) on convertit les quaterrylènetétracarboxodiimides halogénés, par réaction avec des nucléophiles de formule
Z - K
dans laquelle Z est un groupe aryloxy, arylthio, hétéroaryloxy ou hétéroarylthio, dont chacun peut être substitué par des groupes alkyle en C₁-C₄ ou alcoxy en C₁-C₄, et K est le cation d'un métal alcalin ou un atome d'hydrogène, en présence d'une base minérale et d'un composé basique à base d'azote tertiaire, en les quaterrylènetétracarboxodiimides de formule Id dans laquelle Y est Z.

8. Utilisation de quaterrylènetétracarboxodiimides de formule I selon la revendication 1 en tant que colorants fluorescents ou pigments.

9. 9-Halogénopérylène-3,4-dicarboximides de formule générale III 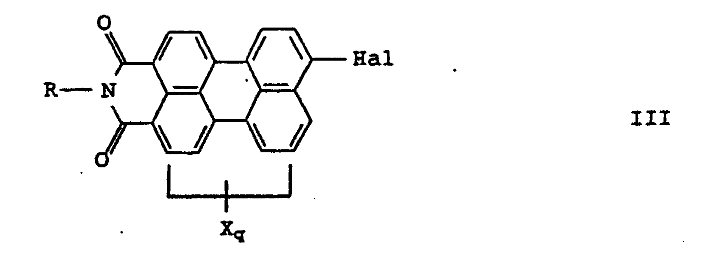 dans laquelle les variables ont les significations suivantes :
R est un atome d'hydrogène ;
un groupe alkyle en C₁-C₃₀, dont la chaîne carbonée peut être interrompue par un ou plusieurs groupements -O-, -S-, -NR¹-, -CO- et/ou -SO₂-, et qui peut être une ou plusieurs fois substitué par des groupes cyano, alcoxy en C₁-C₆, ou par un radical hétérocyclique ayant de 5 à 7 chaînons, lié par l'intermédiaire d'un atome d'azote, qui peut contenir d'autres hétéroatomes et peut être aromatique, où
R¹ est un atome d'hydrogène ou un groupe alkyle en C₁-C₆;
un groupe cycloalkyle en C₅-C₈, dont le squelette carboné peut être interrompu par un ou plusieurs groupements -O-, -S- et/ou -NR¹- ;
un groupe aryle ou hétéroaryle, dont chacun peut être une ou plusieurs fois substitué par des groupes alkyle en C₁-C₁₈, alcoxy en C₁-C₆, cyano, -CONHR², -NHCOR² et/ou aryl- ou hétéroarylazo, dont chacun peut être substitué par des groupes alkyle en C₁-C₁₀, alcoxy en C₁-C₆ ou halogéno, où
R² est un atome d'hydrogène, un groupe alkyle en C₁-C₁₈, aryle ou hétéroaryle dont chacun peut être substitué par des groupes alkyle en C₁-C₆, alcoxy en C₁-C₆, halogéno ou cyano ;
X est un atome d'halogène ; un groupe alkyle en C₁-C₁₈ ; aryloxy, arylthio, hétéroaryloxy ou hétéroarylthio, dont chacun peut être substitué par des groupes alkyle en C₁-C₄ ou alcoxy en C₁-C₄ ;
Hal est un halogène ;
q est un nombre de 2 à 4.
